# EUROPEAN PATENT APPLICATION

(11) **EP 4 736 841 A1**
(43) Date of publication of application: **06.05.2026**
(21) Application number: 24210278.8
(22) Date of filing: 31.10.2024
(51) Int. Cl.: A61K 9/00, A61K 47/32, A61K 9/70

(54) **A MUCOADHESIVE LAYER FOR BUCCAL ADMINISTRATION OF AN ACTIVE PHARMACEUTICAL INGREDIENT**

(71) Applicant: IQ medical GmbH, 82031 Grünwald (DE)
(72) Inventor: CAVELIUS, Christian, 66386 St. Ingbert (DE); HORSTKOTTE, Elke, 82061 Neuried (DE); JOCHEM, Aljosha-Rakim, 66571 Eppelborn (DE); MÜHLHÖLZL-ODÖRFER, Kathrin Ines, 80469 München (DE)
(74) Representative: Engelhard, Markus

(57) **Abstract**

The present invention relates to a mucoadhesive layer for buccal administration of an active pharmaceutical ingredient (API), and to a method of preparing such mucoadhesive layer. Moreover, the present invention also relates to a mucoadhesive film comprising a mucoadhesive layer for buccal administration of an active pharmaceutical ingredient, and to a method of preparing such mucoadhesive film.

## Description

The present invention relates to a mucoadhesive layer for buccal administration of an active pharmaceutical ingredient (API), and to a method of preparing such mucoadhesive layer. Moreover, the present invention also relates to a mucoadhesive film comprising a mucoadhesive layer for buccal administration of an active pharmaceutical ingredient, and to a method of preparing such mucoadhesive film.

### BACKGROUND OF THE INVENTION

The administration of active pharmaceutical ingredients (APIs) is a critical aspect of modern medicine, ensuring that patients receive the correct dosage of medication in an effective manner. Standard administration forms include tablets, capsules, injections and topical creams, each designed to deliver the API efficiently to the target area in the body. Tablets and capsules are widely used for their convenience and ease of use, while injections are often employed for faster delivery of the medication into the blood stream. Topical creams are used for localized treatment, providing direct application to the affected area. Another form of administration that has recently received attention are buccal films which are placed against the inner cheek, allowing the API to be absorbed directly into the blood stream through the mucous membranes. Despite the advancements in these various administration forms, there remain significant challenges to be addressed in ensuring efficient and effective delivery of APIs. One major challenge is the variability in patient response due to difference in metabolism, age, weight and other factors. Additionally, ensuring the stability and bioavailability of APIs in different forms can be complex, requiring sophisticated formulation techniques. There are also issues related to patient compliance, particularly with forms that require precise timing or specific conditions for administration, or with APIs that are hardly soluble or insoluble in water.

Accordingly, it was an object of the present invention to provide for an improved mucoadhesive layer for buccal administration of an active pharmaceutical ingredient. More specifically, it was an object to provide for a mucoadhesive layer for buccal administration, that allows the incorporation of sufficient amounts of active pharmaceutical ingredient (API), whilst, also, ensuring a subsequent efficient release of such API, when the respective mucoadhesive layer has been placed at the intended site of administration, for example in the buccal cavity. Even more specifically, it was an object of the present invention to provide for a mucoadhesive layer allowing for a quantitative release of active pharmaceutical ingredient, preferably of at least 20 wt.% of the total API that had previously been incorporated into the film.

### SUMMARY OF THE INVENTION

All these objects are solved by the present invention, as defined herein in various aspects and embodiments.

In a first aspect, the present invention relates to a mucoadhesive layer for buccal administration of an active pharmaceutical ingredient (API), said layer comprising:
- non-crosslinked linear polyacrylic acid;
- an active pharmaceutical ingredient (API),
- optionally, one or several pharmaceutically acceptable excipient(s).

In one embodiment, said active pharmaceutical ingredient (API) is selected from immunosuppressants, antifungal agents, neurotransmitters, antiasthmatic agents, anti-obesity drugs, nutraceuticals, elastase inhibitors, analgesics, anthelmintics, anti-arrhythmic agents, antibiotics, anticoagulants, antidepressants, antidiabetics, antihistamines, antihypertensive agents, antimuscarinic agents, antimycobacterial agents, antineoplastic agents, antiemetics, antithyroid agents, antiviral agents, anxiolytic sedatives, astringents, beta-adrenoreceptor blocking agents, cardiovascular agents, cardiac inotropic agents, contrast agents, corticosteroids, cough suppressants, diagnostic imaging agents, diuretics, dopaminergics, haemostatics, lipid regulating agents, muscle relaxants, parasympathomimetics, , bisphosphonates, prostaglandins, radiopharmaceuticals, hormones, therapeutic peptides, anti-allergic agents, stimulants, anorectics, sympathomimetics, vasodilatators, antirheumatic agents, antipsychotic agents, and anticonvulsive agents;
In one embodiment, the immunosuppressant is selected from macrolides, corticosteroids, anti-metabolites, non-macrolide calcineurin inhibitors, and other immunosuppressant drugs,
In a preferred embodiment,
- said macrolides are selected from tacrolimus, sirolimus, everolimus and pimecrolimus;
- said corticosteroids are selected from cortisone, prednisone, hydrocortisone, prednisolone, methylprednisolone, dexamethasone, betamethasone, beclomethasone, alclometasone, flumetasone, fluticasone, mometasone, budesonide, ciclesonide, and desonide;
- said antimetabolites are selected from methotrexate, azathioprine, mercaptopurine, and fluorouracil;
- said non-macrolide calcineurin inhibitors are selected from ciclosporin and voclosporin; and
- said other immunosuppressant drugs are selected from fingolimod, TNF-α-binding proteins, such as infliximab, etanercept and adalimumab; mycophenolic acid and its prodrugs, such as mycophenolate mofetil and mycophenolate sodium; and fingolimod.

In one embodiment,
- said antifungal agents are selected from itraconazole, ketoconazole, fluconazole, clotrimazole, miconazole, econazole, tioconazole, posaconazole and voriconazole;
- said neurotransmitters are selected from dopamine, levodopa, carbidopa, adrenaline (ephinephrin), noradrenalin (norepinephrin), acetylcholine, serotonine, glutamate, glycine, gamma-aminobutyric acid (GABA), oxytocin, endorphins, enkephalins, and histamines;
- said antiasthmatic agents are selected from beta-2-agonists, corticosteroids, leukotriene modifiers, mast cell stabilisers, methylxanthines, anticholinergics. biologic agents, and combination medications typically used for inhalation; wherein, preferably, said beta-2-agonists are selected from short-acting beta-agonists (SABAs), e.g. albuterol, or levalbuterol, long-acting beta-agonists (LABAs), e.g. salmeterol, formeterol and vilanterol; said corticosteroids are preferably selected from corticosteroids that are also referred to as "inhaled corticosteroids (ICS)" because of their typical mode of administration, e.g. fluticasone, budesonide, beclomethasone, and mometasone; and corticosteroids that are also referred to as "oral corticosteroids (OCS)" because of their typical mode of administration, e.g. prednisone and prednisolone; said leukotriene modifiers are preferably selected from montelukast, zafirlukast and zileuton; said mast cell stabilizers are preferably selected from cromolyn sodium and nedocromil; said methylxanthines are preferably selected from theophylline and aminophylline; said anticholinergics are preferably selected from short-acting muscarinic antagonists (SAMAs), e.g. ipratropium bromide, and long-acting muscarinic antagonists (LAMAs), e.g. tiotropium or umeclidinium; said biologic agents are preferably selected from omalizumab, mepolizumab, benralizumab and dupilumab; and wherein said combination medication is preferably selected from combinations of inhaled corticosteroid(ICS)/long-acting beta agonist(LABA), e.g. fluticasone/salmeterol or budesonide/formoterol, and combinations of inhaled corticosteroid(ICS)/long-acting beta agonist(LABA)/long-acting muscarinic antagonists (LAMAs), e.g. fluticasone/vilanterol/umeclidinium;
- said anti-obesity drugs are selected from appetite suppressants. Lipase inhibitors, GLP-1 receptor agonists, serotonin 2C agonists, and sympathomimetic amines; wherein, preferably, said appetite suppressants are selected from phentermine, diethylpropion, and combinations of phentermine and topiramate; wherein, preferably, said lipase inhibitors are selected from orlistat, cetilistat, and lipstatin; wherein, preferably, said GLP-1 receptor agonists are selected from liraglutide and semaglutide; wherein, preferably, said serotonin 2C agonists are selected from lorcaserin, vabicaserin, and WAY-163909 ((7bR,10aR)-2,3,4,7b,8,9,10,10a-Octahydro-1H-cyclopenta[b][1,4]diazepino[6,7,1-hi]indole); wherein, preferably, said sympathomimetic amines are selected from benzphetamine and phendimetrazine;
- said nutraceuticals are selected from vitamins, minerals, omega-3 fatty acids, omega-6 fatty acids, herbal supplements, probiotics, prebiotics, amino acid supplements, protein supplements, antioxidants, fibre supplements, botanical extracts, and specialty nutrients; wherein, preferably, said vitamins are selected from vitamins D, C, A, K, B6, B12, E, niacin, folate, riboflavin, pantothenic acid and biotin; wherein, preferably said minerals are selected from calcium, magnesium, zinc, iron, selenium, potassium, copper, chromium, and iodine; wherein, preferably, said omega-3 fatty acids are selected from eicosapentaenoic acid (EPA) and docosahexaenoic acid (DHA); wherein, preferably, said omega-6 fatty acids are selected from linoleic acid (LA), gamma-linolenic acid (GLA), dihomo-gamma-linolenic acid (DGLA), and arachidonic acid (AA); wherein, preferably, said herbal supplements are selected from Echinacea, Gingko Biloba, Turmeric, and Ginseng; wherein, preferably, said probiotics are selected from Lactobacillus strains and Bifidobacterium strains that support gut health; wherein, preferably, said prebiotics are selected from non-digestible fibres such as inulin and fructooligosaccharides (FOS) that promote the growth of beneficial gut bacteria; wherein, preferably, said amino acid supplements are selected from branched-chain amino acids (BCAA), e.g. leucine, isoleucine, and valine, essential amino acids (EAA), such as methionine, phenylalanine, threonine, tryptophan, lysine, and histidine, and conditionally essential amino acids, such as glutamine, arginine, and tyrosine; wherein, preferably, said proein supplements are selected from whey protein, casein protein., soy protein, pea protein, hemp protein, rice protein, and collagen protein; wherein, preferably, said antioxidants are selected from vitamins C and E, selenium, zin, beta-carotene, lycopene, lutein, zeaxanthin, resveratrol, quercetin, green tea extract, epigallocatechin gallate (EGCG), curcumin, anthocyanins, Coenzyme Q10 (CoQ10), alpha-lipoic acid (ALA) and glutathione; wherein, preferably, said fibre supplements are selected from psyllium husk, inulin, beta-glucan, glucomannan, wheat bran, cellulose, acacia fibre, apple fibre, fructooligosaccharides (FOS), galactooligosaccharides (GOS), chia seeds, flaxseeds, plantago ovata seeds and husk, and resistant starch; wherein, preferably, said botanical extracts are selected from green tea extract, chamomile, hibiscus, cranberry, elderberry, grape seed extract, acai berry, Echinacea, Ginseng, Gingko biloba, turmeric, milk thistle, saw palmetto, pycnogenol and black cumin seed; wherein, preferably, said specialty nutrients are selected from glucosamine, chondroitin, lutein, zeaxanthin, D-ribose, trehalose, N-acetyl cysteine (NAC), phosphatidylserine, astaxanthin, dehydroepiandrosterone (DHEA), and melatonin;
- said elastase inhibitors are selected from alpha-1 antitrypsin (AAT), sivelestat, elafin, AZD9668, ONO-5046, serpina1, peptide-based elastase inhibitors, small molecule elastase inhibitors, epigallocatechin gallate (EGCG), and diosmin;
- said analgesics are selected from non-opioid analgesics, nonsteroidal anti-inflammatory drugs (NSAIDs), opioid analgesics, adjuvant analgesics, topical analgesics, and combination analgesics; wherein, preferably, said non-opioid analgesics are selected from acetaminophen, capsaicin, lidocaine, menthol, acetylcysteine, and ketorolac; wherein, preferably, said nonsteroidal anti-inflammatory drugs (NSAIDs) are selected from acetylsalicylic acid, ibuprofen, naproxen, diclofenac, and celecoxib; wherein, preferably, said opioid analgesics are selected from morphine, oxycodone, hydrocodone, fentanyl, codein and tramadol; wherein, preferably, said adjuvant analgesics are selected from antidepressants, e.g. amitriptyline and duloxetine; anticonvulsants, e.g. gabapentin and pregabalin; and muscle releaxants, e.g. cyclobenzaprine and baclofen; wherein, preferably said topical analgesics are selected from capsaicin, lidocaine, and diclofenac; wherein, preferably, said combination analgesics are selected from Acetaminophen/Codeine, Acetaminophen/Oxycodone, and Acetaminophen/Hydrocodone;
- said anthelmintics are selected from benzimidazoles, e.g. albendazole, mebendazole, or thiabendazole; avermectins, e.g. ivermectin; pyrantel pamoate, praziquantel, diethyl carbamazine, niclosamide, levamisole, piperazine, oxamniquine, and bithionol;
- said anti-arrhythmic agents are selected from sodium channel blockers, beta blockers, potassium channel blockers, calcium channel blockers, and other anti-arrhythmic agents not falling under any of the aforementioned blockers and being selected from adenosine, digoxine, and magnesium sulfate; wherein, preferably, said sodium channel blockers are selected from Quinidine, Procainamide, Disopyramide, Lidocaine, Mexiletine, Phenytoin, Flecainide, and Propafenone; wherein, preferably, said beta blockers are selected from Propranolol, Metoprolol, Atenolol, and Esmolol; wherein, preferably, said potassium channel blockers are selected from amiodarone, sotalol. dofetilide, ibutilide, and dronedarone; wherein, preferably, said calcium channel blockers are selected from verapamil, diltiazem, amlodipine, nifedipine, felodipine, nicardipine, isradipine, nimodipine, clevidipine, lercanidipine, and lacidipine;
- said antibiotics are selected from beta-lactams, macrolides, quinolones/fluoroquinolones, aminoglycosides, tetracyclines, sulfonamides, glycopeptides, oxazolidinones, lincosamides, nitroimidazoles, polypeptide antibiotics, rifamycins, and other antibiotics not falling under any of the aforementioned antibiotic classes and being selected from chloramphenicol, daptomycin, fosfomycin, and nitrofurantoin; wherein, preferably, said beta-lactams are selected from penicillin, in particular penicillin G, amoxicillin, ampicillin, oxacillin, piperacillin, cefazolin, cephalexin, cefuroxime, ceftriaxone, cefepime, iminopenem, meropenem, ertapenem, doripenem, and aztreonam; wherein, preferably, said macrolides are selected from erythromycin,, azithromycin, and clarithromycin; wherein, preferably, said quinolones/fluoroquinolones are selected from ciprofloxacin, levofloxacin, moxifloxacin, ofloxacin, norfloxacin, Gemifloxacin, gatifloxacin, lomefloxacin, sparfloxacin, trovafloxacin, enoxacin and prulifloxacin; wherein, preferably, said aminoglycosides are selected from gentamicin, tobramycin, amikacin, and streptomycin; wherein, preferably, said tetracyclines are selected from tetracycline, doxycycline, and minocycline; wherein, preferably, said sulfonamides are selected from sulfamethoxazole, sulfadiazine, sulfisoxazole, sulfapyridine, trimethoprim-sulfamethoxazole, and sulfadoxine-pyrimethamine; wherein, preferably, said glycopeptides are selected from vancomycin, and teicoplanin; wherein, preferably, said oxazolidinones are selected from linezolid, tedizolid, radezolid, contezolid, and sutezolid; wherein, preferably, said lincosamides are selected from clindamycin and lincomycin; wherein, preferably, said nitroimidazoles are selected from metronidazole, tinidazole, secnidazole, ornidazole, and nimorazole; wherein, preferably, said polypeptide antibiotics are selected from polymyxin B, polymyxin E, gramicidin, tyrothricin, and vancomycin; wherein, preferably, said rifamycines are selected from rifampin, and rifabutin;
- said anticoagulants are selected from vitamin K antagonists, direct oral anticoagulants, heparins, synthetic pentasaccharides, parenteral direct thrombin inhibitors, coumarins, and other anticoagulants not falling under any of the aforementioned anticoagulant classes and being selected from danaparoid and desirudin; wherein, preferably, said vitamin K antagonists are selected from warfarin, acenocoumarol, and phenprocoumon; wherein, preferably, said direct oral anticoagulants are selected from dabigatran, rivaroxaban, apixaban, edoxaban, and betrixaban; wherein, preferably, said heparins are selected from unfractionated heparin, enoxaparin, dalteparin, and tinzaparin; wherein, preferably, said synthetic pentasaccharides are selected from fondaparinux, idraparinux, and idrabiotaparinux; wherein, preferably, said parenteral direct thrombin inhibitors are selected from bivalirudine and argatroban; wherein, preferably, said coumarins are selected from acenocoumarol, and phenprocoumon;
- said antidepressants are selected from selective serotonin reuptake inhibitors (SSRIs), serotonin-norepinephrine reuptake inhibitors (SNRIs), tricyclic antidepressants (TCAs), monoamine oxidase inhibitors, atypical antidepressants, serotonin modulators, noradrenergic and specific serotonergic antidepressants (NaSSAs), and serotonin antagonist and reuptake inhibitors; wherein, preferably, said selective serotonin reuptake inhibitors (SSRIs) are selected from fluoxetine, sertraline, citalopram, escitalopram, paroxetine, and fluvoxamine; wherein, preferably, said serotonin-norepinephrine reuptake inhibitors (SNRIs) are selected from venlafaxine, duloxetine, desvenlafaxine, and levomilnacipran; wherein, preferably, said tricyclic antidepressants (TCA) are selected from amitriptyline, nortriptyline, imipramine, desipramine, clomipramine, doxepin, and trimipramione; wherein, preferably, said monoamine oxidase inhibitors are selected from phenelzine, tranylcypromine, isocarboxazide, and selegiline; wherein, preferably, said atypical antidepressants are selected from bupropion, mirtazapine, trazodone, and nefazodone; wherein, preferably, said serotonin modulators are selected from vilazodone and vortioxetine; wherein, preferably, said noradrenergic and specific serotonergic antidepressants (NaSSAs) are selected from mirtazapine, and mianserin; wherein, preferably, said serotonin antagonist and reuptake inhibitors are selected from trazodone, and nefazodone;
- said antidiabetics are selected from insulins, biguanides, sulfonylureas, meglitinides, thiazolidinediones (TZDs), dipeptidyl peptidase-4 (DPP-4) inhibitors, glucagon-like peptide-1 (GLP-1) receptor agonists, sodium-glucose co-transporter-2 (SGLT2) inhibitors, alpha-glucosidase inhibitors, amylin analogues, bile acid sequestrants, and dopamine agonists; wherein, preferably, said insulins are selected from insulin lispro, insulin aspart, insulin glulisine, regular insulin, NPH insulin, insulin glargine, insulin detemir, and insulin degludec; wherein, preferably, said biguanides are selected from metformin, phenformin and buformin; wherein, preferably, said sulfonylureas are selected from glipizide, glyburide, and glimepiride; wherein, preferably, said meglitinides are selected from repaglinide, and nateglinide; wherein, preferably, said thiazolidinediones are selected from pioglitazone, and rosiglitazone; wherein, preferably, said dipeptidyl peptidase-4 (DPP-4) inhibitors are selected from sitagliptin, saxagliptin, linagliptin,, and alogliptin; wherein, preferably, said glucagon-like peptide-1 (GLP-1) receptor agonists are selected from exenatide, liraglutide, dulaglutide, semaglutide, and lixisenatide; wherein, preferably, said sodium-glucose co-transporter-2 (SGLT2) inhibitors are selected from canagliflozin, dapagliflozin, empagliflozin, and ertugliflozin; wherein, preferably, said alpha-glucosidase inhibitors are selected from acarbose, and miglitol; wherein, preferably, said amylin analogues are pramlintide; wherein, preferably, said bile-acid sequestrants are selected from colesevelam, cholestyramine, and colestipol; wherein, preferably, said dopamine agonists are selected from bromocriptine, cabergoline, pramipexole, ropinirole, rotigotine, apomorphine, piribedil, and quinagolide;
- said antihistamines are selected from Diphenhydramine, Chlorpheniramine (Chlor-Trimeton), Brompheniramine, Clemastine, Hydroxyzine, Promethazine, Doxylamine, Meclizine, Dimenhydrinate, Loratadine, Cetirizine, Fexofenadine, Desloratadine, Levocetirizine, Bilastine, Rupatadine, Olopatadine, Azelastine, and Ketotifen;
- said antihypertensive agents are selected from diuretics, beta blockers, angiotensin-converting enzyme (ACE) inhibitors, angiotensin II receptor blockers (ARBs), calcium channel blockers (CCBs), alpha blockers, centrally acting antihypertensive agents, direct vasodilators, and renin inhibitors; wherein, preferably, said diuretics are selected from hydrochlorothiazide, chlorthalidone, indapamide, metolazone, furosemide, bumetamide, torsemide, spironolactone, eplerenone, amiloride, and triamterene; wherein, preferably, said beta blockers are selected from Propranolol, nadolol, metoprolol, atenolol, bisoprolol, Esmolol, carvedilol, and labetalol; wherein, preferably, said angiotensin-converting enzyme (ACE) inhibitors are selected from lisinopril, enalapril, ramipril, benazepril, quinapril, and captopril; wherein, preferably, said angiotensin II receptor blockers (ARBs) are selected from losartan, valsartan, Olmesartan, irbesartan, telmisartan, and candesartan; wherein, preferably, said calcium channel blockers (CCBs) are selected from amlodipine, nifedipine, felodipine, nicardipine, verapamil, and diltiazem; wherein, preferably, said alpha blockers are selected from prazosin, terazosin, and doxazosin; wherein, preferably, said centrally acting antihypertensive agents are selected from clonidine, methyldopa, and guanfacine; wherein, preferably, said direct vasodilators are selected from hydralazine, and minoxidil; wherein, preferably, said renin inhibitors are selected from aliskiren, remikiren, enalkiren, and zankiren;
- said antimuscarinic agents are selected from Oxybutynin, Tolterodine, Solifenacin, Darifenacin, Trospium, Fesoterodine, Ipratropium, Tiotropium, Aclidinium, Umeclidinium, Hyoscyamine, Dicyclomine, Glycopyrrolate, Scopolamine, Benztropine, Trihexyphenidyl, Atropine, Tropicamide, Cyclopentolate;
- said antimycobacterial agents are selected from Isoniazid, Rifampin, Ethambutol, Pyrazinamide, Rifabutin, Rifapentine, Streptomycin, Amikacin, Kanamycin, Capreomycin, Ethionamide, Prothionamide, Cycloserine, Para-aminosalicylic acid (PAS), Levofloxacin, Moxifloxacin, Ciprofloxacin, Bedaquiline, Delamanid, Linezolid, Dapsone, Rifampin, Clofazimine, Clarithromycin, Azithromycin, Amikacin, Ethambutol, Rifabutin, Rifampin, Ciprofloxacin, Levofloxacin, Moxifloxacin, Linezolid
- said antineoplastic agents are selected from alkylating agents, antimetabolites, natural product antineoplastic agents, antitumor antibiotics, hormonal antineoplastic agents, monoclonal antibodies, tyrosine kinase inhibitors, checkpoint inhibitors, platinum compounds, proteasome inhibitors, and immunomodulatory agents; wherein, preferably, said alkylating agents are selected from cyclophosphamide, ifosfamide, melphalan, and chlorambucil; wherein, preferably, said antimetabolites are selected from methotrexate, 5-fluoruracil (5-FU), cytarabine, and gemcitabine; wherein, preferably, said natural product antineoplastic agents are selected from vinca alkaloids, e.g. vincristine, vinblastine; taxanes, e.g. paclitaxel, docetaxel; epipodophyllotoxins, e.g. etoposide, teniposide; camptothecins, e.g. irinotecan, topotecan; wherein, preferably, said antitumor antibiotics are selected from doxorubicin, daunorubicin, bleomycin, and mitomycin; wherein, preferably, said hormonal antineoplastic agents are selected from tamoxifen, anastrozole, letrozole, and leuprolide; wherein, preferably, said monoclonal antibodies are selected from rituximab, trastuzumab, bevacizumab, and pembrolizumab; wherein, preferably, said tyrosine kinase inhibitors are selected from imatinib, erlotinib, sunitinib, and Lenvatinib; wherein, preferably, said checkpoint inhibitors are selected from pembrolizumab, nivolumab, atezolizumab, durvalumab, avelumab, and ipilimumab; wherein, preferably, said platinum compounds are selected from cisplatin, carboplatin, oxaliplatin; wherein, preferably, said proteasome inhibitors are selected from bortezomib, carfilzomib, and ixazomib; wherein, preferably, said immunomodulatory agents are selected from thalidomide and lenalidomide;
- said antiemetics are selected from serotonin (5-HT3) receptor antagonists, dopamine antagonists, NK1 receptor antagonists, antihistamines, anticholinergics, benzodiazepines, cannabinoids, and steroids; wherein, preferably, said serotonin (5-HT3) receptor antagonists are selected from ondansetron, granisetron, dolasetron, and palonosetron; wherein, preferably, said dopamine antagonists are selected from metoclopramide, prochlorperazine, and haloperidol; wherein, preferably, said NK1 receptor antagonists are selected from aprepitant, fosaprepitant, and rolapitant; wherein, preferably, said antihistamines are selected from diphenhydramine, meclizine, and promethazine; wherein, preferably, said anticholinergics are scopolamine; wherein, preferably, said benzodiazepines are selected from lorazepam, and diazepam; wherein, preferably, said cannabinoids are selected from dronabinol, and nabilone; wherein, preferably, said steroids are selected from dexamethasone;
- said antithyroid agents are selected from thionamides, e.g. methimazole, propylthiouracil (PTU); iodine solution, and radioactive iodine;
- said antiviral agents are selected from nucleoside and nucleotide analogues, protease inhibitors, non-nucleoside reverse transcriptase inhibitors (NNRTIs), integrase inhibitors, fusion inhibitors, neuraminidase inhibitors, polymerase inhibitors, entry inhibitors, small molecule antiviral agents not falling under any of the aforementioned antiviral agent classes; wherein, preferably, said nucleoside and nucleotide analogues are selected from acyclovir, valacyclovir, ganciclovir, valganciclovir, tenofovir, lamivudine, and ribavirin; wherein, preferably, said protease inhibitors are selected from ritonavir, lopinavir, simeprevir, and grazoprevir; wherein, preferably, said non-nucleoside reverse transcriptase inhibitors (NNRTIs) are selected from efavirent, nevirapine, and etravirine; wherein, preferably, said integrase inhibitors are selected from raltegravir, dolutegravir, and elvitegravir; wherein, preferably, said fusion inhibitors are selected from enfuvirtide and albuvirtide; wherein, preferably, said neuraminidase inhibitors are selected from oseltamivir, and zanamivir; wherein, preferably, said polymerase inhibitors are selected from sofosbuvir, and remdesivir; wherein, preferably, said entry inhibitors are selected from maraviroc and ibalizumab; wherein, preferably, said small molecule antiviral agents are selected from ribavirin and foscarnet;
- said anxiolytic sedatives are selected from benzodiazepines, non-benzodiazepine anxiolytics, barbiturates, antihistamines, beta blockers, selective serotonin reuptake inhibitors (SSRIs), serotonin-norepinephrine reuptake inhibitors (SNRIs), and tricyclic antidepressants; wherein, preferably, said benzodiazepines are selected from diazepam, lorazepam, alprazolam, clonazepam, chlordiazepoxide, temazepam, and midazolam; wherein, preferably, said non-benzodiazepine anxiolytics are selected from buspirone; wherein, preferably, said barbiturates are selected from phenobarbital, and secobarbital; wherein, preferably, said antihistamines are selected from hydroxyzine, and diphenhydramine; wherein, preferably, said beta-blockers are selected from propranolol, and atenolol; wherein, preferably, said selective serotonine reuptake inhibitors (SSRIs) are selected from sertraline, paroxetine, and fluoxetine; wherein, preferably, said serotonin-norepinephrine reuptake inhibitors (SNRIs) are selected from venlafaxine, and duloxetine; wherein, preferably, said tricyclic antidepressants are selected from amitriptyline and imipramine;
- said astringents are selected from astringent aluminium compounds, witch hazel extract, tannins, calamine, astringent zinc compounds, astringent silver compounds, astringent alcohols, astringent acids, and astringent herbal agents;
- said beta-adrenoreceptor blocking agents are selected from non-selective beta-blockers, selective beta-blockers, beta-blockers with intrinsic sympathomimetic activity (ISA), and beta-blockers with alpha-blocking activity; wherein, preferably, said non-selective beta-blockers are selected from propranolol, nadolol, timolol, and sotalol; wherein, preferably, said selective beta-blockers are selected from atenolol, metoprolol, bisoprolol, esmolol, and nebivolol; wherein, preferably, said beta-blockers with intrinsic sympathomimetic activity (ISA) are selected from pindolol, acebutolol, and carteolol; wherein, preferably, said beta-blockers with alpha-blocking activity are selected from carvedilol, and labetalol;
- said cardiovascular agents are selected from antihypertensive agents, antiarrhythmic agents, anticoagulants, antiplatelet agents, lipid-lowering agents, vasodilators, cardiac glycosides, inotropes, angiotensin receptor-neprilysin inhibitors (ARNIs), and aldosterone antagonists; wherein, preferably, said antihypertensive agents are selected from ACE inhibitors, e.g. lisinopril, enalapril, and ramipril; Angiotensin II receptor blockers (ARBs), e.g. losartan, valsartan, and irbesartan; beta-blockers, e.g. metoprolol, atenolol, and carvedilol; calcium channel blockers, e.g. amlodipine, diltiazem, and verapamil; and diuretics, e.g. hydrochlorothiazide, furosemide, and spironolacton; wherein, preferably, said antiarrhythmic agents are selected from Class I (Sodium Channel Blockers), e.g. Quinidine, Procainamide, Lidocaine; Class II (Beta-Blockers), e.g. Propranolol, Esmolol; Class III (Potassium Channel Blockers), e.g. Amiodarone, Sotalol; and Class IV (Calcium Channel Blockers), e.g. Verapamil, Diltiazem; wherein, preferably, said anticoagulants are selected from warfarin, heparin, enoxaparin, dabigatran, rivaroxaban, and apixaban; wherein, preferably, said antiplatelet agents are selected from acetylsalicylic acid, clopidogrel, prasugrel, and ticagrelor; wherein, preferably, said lipid lowering agents are selected from statins, e.g. atorvastatin, simvastatin, rosuvastatin; fibrates, e.g. gemfibrozil, and fenofibrate; bile acid sequestrants, e.g. cholestyramine, and colestipol; and PCSK9 inhibitors, e.g. alirocumab, and evolocumab; wherein, preferably, said vasodilators are selected from nitroglycerin, isosorbide dinitrate, hydralazine, and minoxidil; wherein, preferably, said cardiac glycosides are selected from digoxin, and digitoxin; wherein, preferably, said inotropes are selected from dobutamine, and milrinone; wherein, preferably, said angiotensin receptor-neprilysin inhibitors (ARNIs) are selected from sacubitril/valsartan; wherein, preferably, said aldosterone antagonists are selected from spironolactone, and eplerenone;
- said cardiac inotropic agents are selected from cardiac glycosides, sympathomimetic amines, phosphodiesterase inhibitors, calcium sensitizers, and beta-adrenergic agonists; wherein, preferably, said cardiac glycosides are selected from digoxin, and digitoxin; wherein, preferably, said sympathomimetic amines are selected from dobutamine, dopamine, epinephrine, and norepinephrine; wherein, preferably, said phosphodiesterase inhibitors are selected from milrinone, and inamrinone; wherein, preferably, said calcium sensitizers are selected from levosimendan, and pimobendan; wherein, preferably, said beta-adrenergic agonists are selected from isoproterenol, and dobutamine;
- said contrast agents are selected from iodinated contrast agents, gadolinium-based contrast agents, barium sulfate, microbubble contrast agents, and iron oxide nanoparticles; wherein, preferably, said iodinated contrast agents are selected from iohexol, iopamidol, iodixanol, ioversol, and iopromide; wherein, preferably, said gadolinium-based contrast agents are selected from gadopentetate dimeglumine, gadobutrol, gadoteridol, gadodiamide, and gadoxetate disodium; wherein, preferably, said barium sulfate is a barium sulfate suspension; wherein, preferably, said microbubble contrast agents are selected from perflutren lipid microspheres (Definity^{®}), perflutren protein-type A microspheres (Optison^{®}), and sulfur hexafluoride microbubbles (SonoVue^{®});
- said corticosteroids are selected from glucocorticoids and mineralocorticoids; wherein, preferably, said glucocorticoids are selected from prednisone, prednisolone, methylprednisolone, dexamethasone, hydrocortisone, betamethasone, and triamcinolone; wherein, preferably, said mineralocorticosteroids are selected from aldosterone, fludrocortisone, and deoxycorticosterone;
- said cough suppressants are selected from opioid derivatives, non-opioid cough suppressants, and peripheral acting antitussive agents; wherein, preferably, said opioid derivatives are selected from codeine, and hydrocodone; wherein, preferably, said non-opioid cough suppressants are selected from dextromethorphan, benzonatate, diphenhydramine, chlophedianol, levocloperastine, and butamirate; wherein, preferably, said peripheral acting antitussive agents are selected from benzonatate, butamirate, levodropropizine, and oxolamine
- said diagnostic imaging agents are selected from iodinated contrast media, gadolinium-based contrast agents, barium sulfate, microbubble contrast agents, radiopharmaceuticals, and fluorescent dyes; wherein, preferably, said iodinated contrast agents, said gadolinium-based contrast agents, said barium sulfate, and said microbubble contrast agents are as defined further above in the context contrast agents; wherein, preferably, said radiopharmaceuticals are selected from Technetium-99m-based, Fluorine-18-based, Iodine-123-based, Gallium-67-based, and Thallium-201-based radiopharmaceuticals; and wherein, preferably, said fluorescent dyes are selected from fluorescein and indocyanine green;
- said diuretics are selected from thiazide diuretics, loop diuretics, potassium-sparing diuretics, carbonic anhydrase inhibitors, and osmotic diuretics; wherein, preferably, said thiazide diuretics are selected from hydrochlorothiazide, chlorthalidone, indapamide, and metolazone; wherein, preferably, said loop diuretics are selected from furosemide, bumetanide, torsemide, and ethacrynic acid; wherein, preferably, said potassium-sparing diuretics are selected from spironolactone, eplerenone, amiloride, and triamterene; wherein, preferably, said carbonic anhydrase inhibitors are selected from acetazolamide, and methazolamide; wherein, preferably, said osmotic diuretics are selected from mannitol, and glycerol
- said dopaminergics are selected from dopamine precursors, dopamine agonists, monoamine oxidase-B (MAO-B) inhibitors, catechol-O-methyltransferase (COMT) inhibitors, dopamine releasing agents, and dopamine reuptake inhibitors; wherein, preferably, said dopamine precursors are selected from levodopa, L-phenylalanine, and L-tyrosine; wherein, preferably, said dopamine agonists are selected from pramipexole, ropinirole, bromocriptine, cabergoline, pergolide, and apomorphine; wherein, preferably, said monoamine oxidase-B (MAO-B) inhibitors are selected from selegiline, rasagiline, and safinamide; wherein, preferably, said catechol-O-methyltransferase (COMT) inhibitors are selected from entacapone, and tolcapone; wherein, preferably, said dopamine releasing agents are selected from amantadine, methamphetamine, methylphenidate, dexamphetamine, and modafinil; wherein, preferably, said dopamine reuptake inhibitors are selected from bupropion, methylphenidate, cocaine, nomifensine, and amineptine;
- said hemostatics are selected from systemic hemostatics selected from antifibrinolytics, tranexamic acid, aminocaproic acid, vitamin K, phytonadione, desmopressin; topical hemostatics selected from gelatin sponges, oxidized cellulose, microfibrillar collagen, thrombin, fibrin, chitosan; and hemostatic dressing and agents selected from kaolin and zeolite;
- said lipid regulating agents are selected from statins, fibrates, bile acid sequestrants, niacin, cholesterol absorption inhibitors, omega-3 fatty acid ethyl esters, PCSK9 inhibitors and ATP citrate lyase (ACL) inhibitors; wherein, preferably, said statins are selected from atorvastatin, simvastatin, rosuvastatin, and lovastatin; wherein, preferably, said fibrates are selected from fenofibrate, and gemfibrozil; wherein, preferably, said bile acid sequestrants are selected from cholestyramine, colestipol, and colesevelam; wherein, preferably, said cholesterol absorption inhibitors are selected from ezetimibe, bempedoic acid, plant sterols, and plant stanols; wherein, preferably, said omega-3 fatty acid ethyl esters are selected from eicosapentaenoic acid (EPA) ethyl esters and docosahexaenoic acid (DHA) ethyl esters; wherein, preferably, said PCSK9 inhibitors are selected from alirocumab, and evolocumab; wherein, preferably, said ATP citrate lyase (ACL) inhibitors are selected from bempedoic acid ;
- said muscle relaxants are selected from baclofen, cyclobenzaprine, methocarbamol, tizanidine, diazepam, carisoprodol, metaxalone, orphenadrine, and chlorzoxazone;
- said parasympathomimetics are selected from bethanechol, pilocarpine, carbachol, methacholine, cevimeline, neostigmine, pyridostigmine, physostigmine, edrophonium and rivastigmine;
- said bisphosphonates are selected from alendronate, risedronate, ibandronate, soledronate, etidronate and pamidronate;
- said prostaglandins are selected from prostaglandin E1 (PGE1, alprostadil), prostaglandin E2 (PGE2, dinoprostone), prostaglandin F2α (PGF2α, dinoprost), carboprost, misoprostol, latanoprost, travoprost, bimatoprost, and iloprost;
- said radiopharmaceuticals are selected from diagnostic and therapeutic radiopharmaceuticals, wherein said diagnostic radiopharmaceuticals are selected from Technetium-99m-based, Fluorine-18-based, Iodine-123-based, Gallium-67-based and Indium-111-based radiopharmaceuticals; and wherein said therapeutic radiopharmaceuticals are selected from Iodine-131-based, Yttrium -go-based, Samarium-153-based, Lutetium-177-based, and Radium-223-based radiopharmaceuticals;
- said hormones are selected from non-steroid hormones and steroid hormones; wherein, preferably, said non-steroid hormones are selected from insulin, glucagon, thyroxine (T4), triiodothyronine (T3), parathyroid hormone (PTH), calcitonin, growth hormone (GH), prolactin, adrenocorticotropic hormone (ACTH), follicle-stimulating hormone (FSH), luteinizing hormone, oxytocin, and antidiuretic hormone (ADH); and wherein, preferably, said steroid hormones are selected from cortisol, aldosterone, testosterone, estradiol, progesterone, dehydroepiandrosterone (DHEA), estrone, and estriol;
- said anti-allergic agents are selected from antihistamines, mast cell stabilizers, corticosteroids, leukotriene receptor antagonists, immunomodulators, and degongestants; wherein, preferably, said antihistamines are selected from diphenhydramine, chlorpheniramine, loratadine, cetirizine, fexofenadine, desloratadine, and levocetirizine; wherein, preferably, said mast cell stabilizers are selected from cromolyn sodium, and nedocromil; wherein, preferably, said corticosteroids are selected from prednisone, methylprednisolone, fluticasone, budesonide, beclomethasone, and triamcinolone; wherein, preferably, said leukotriene receptor antagonists are selected from montelukast, and zafirlukast; wherein, preferably, said immunomodulators are selected from omalizumab, and dupilumab; wherein, preferably, said decongestants are selected from pseudoephedrine, and phenylephrine;
- said stimulants are selected from amphetamines, methylphenidates, xanthines, norepinephrine uptake inhibitors, modafinil-compounds, cocaine, ephedrine, and pseudoephedrine; wherein, preferably, said amphetamines are selected from amphetamine, dextroamphetamine, ands lisdexamfetamine; wherein, preferably, said mehtylphenidates are selected from methylphenidate, and dexmethylphenidate; wherein, preferably, said xanthines are selected from caffeine, and theophylline; wherein, preferably, said norepinephrine uptake inhibitors are selected from atomoxetine reboxetine, viloxazine, paprotiline, desipramine, and nortriptyline; wherein, preferably, said modafinil compounds are selected from modafinil, and armodafinil;
- said anorectics are selected from sympathomimetic anorectic compounds, serotonin-norepinephrine reuptake inhibitors (SNRIs), serotonin receptor agonists, GLP-1 receptor agonists, combination medications of stimulants and anticonvulsants or of antidepressants and opioid antagonists, and orlistat, wherein said combination medications are selected from the combinations phentermine/topiramate and bupropion/naltroxone; wherein, preferably, said sympathomimetic anorectics are selected from phentermine, diethylpropion, benzphetamine, and phendimetrazine; wherein, preferably, said serotonin-norepinephrin reuptake inhibitors (SNRIs) are selected from silbutramine, venlafaxine, duloxetine, desvenlafaxine, milnacipran, levomilnacipran, and atomoxetine; wherein, preferably, said GLP-1 receptor agonists are selected from liraglutide, and semaglutide; wherein, preferably, said combination medications of stimulants and anticonvulsants or of antidepressants and opioid antagonists are selected from phentermine/topiramate, and bupropion/naltrexone;
- said sympathomimetics are selected from direct-acting sympathomimetics, indirect-acting sympathomimetics, mixed-acting sympathomimetics, beta-agonists, and alpha-agonists, wherein, preferably, said direct-acting sympathomimetics are selected from epinephrine, norepinephrine, dopamine, phenylephrine, dobutamine, and isoproterenol; said indirect-acting sympathomimetics are, preferably, selected from amphetamine, methamphetamine, ephedrine and pseudoephedrine; said mixed-acting sympathomimetics are, preferably, selected from ephedrine and metaraminol; said beta-agonists are, preferably, selected from albuterol, salmeterol, formoterol and terbutaline; and said alpha-agonists are, preferably, selected from clonidine, methyldopa and oxymetazoline;
- said vasodilatators are selected from nitrate compounds, calcium channel blockers, angiotensin-converting enzyme (ACE) inhibitors, angiotensin II receptor blockers (ARBs), direct vasodilators, phosphodiesterase inhibitors, potassium channel openers, endothelin receptor antagonists, and prostacyclin analogues; wherein, preferably, said nitrate compounds are selected from nitroglycerin, isosorbide mononitrate, and isosorbide dinitrate; said calcium channel blockers are, preferably, selected from amlodipine, nifedipine, verapamil, diltiazem; said angiotensin-converting enzyme (ACE) inhibitors are, preferably, selected from enalapril, lisinopril, and ramipril; said angiotensin II receptor blockers (ARBs) are, preferably, selected from losartan, valsartan, and candesartan; said direct vasodilators are, preferably, selected from hydralazine and minoxidil; said phosphodiesterase inhibitors are, preferably, selected from sildenafil, tadalafil, and milrinone; said potassium channel openers are, preferably, selected from nicorandil and diazoxide; said endothelin receptor antagonists are, preferably, selected from bosentan, and ambrisentan; and said prostacyclin-analogues are, preferably, selected from epoprostenol, iloprost and treprostinil;
- said antirheumatic agents are selected from small molecule disease-modifying antirheumatic drugs (DMARDs), biologic disease-modifying antirheumatic drugs (DMARDs), and targeted synthetic disease-modifying antirheumatic drugs (DMARDs); wherein said small molecule disease-modifying antirheumatic drugs (DMARDs) are, preferably, selected from methotrexate, sulfasalazine, hydroxychloroquine, leflunomide, azathioprine, cyclosporine, and cyclophosphamide; said biologic disease-modifying antirheumatic drugs (DMARDs) are, preferably, selected from TNF-α inhibitors, B-cell inhibitors, T-cell co-stimulation modulators and interleukin inhibitors; and wherein said targeted synthetic disease-modifying antirheumatic drugs (DMARDs) are, preferably, selected from janus kinase inhibitors; wherein, more preferably, said TNF-α inhibitors are selected from adalimumab, etanercept, infliximab, golimumab, certolizumab, and certolizumab pegol; wherein, more preferably, said B-cell inhibitors are selected from rituximab, obinutuzumab, ofatumumab, ibritumomab tiuxetane, belimumab, and ocrelizumab; wherein, more preferably, said T-cell co-stimulation modulators are selected from abatacept, belatacept, alefacept, and teplizumab; wherein, more preferably, said interleukin inhibitors are selected from tocilizumab, anakinra and secukinumab; wherein, more preferably, said janus kinase inhibitors are selected from tofacitinib, baricitinib, and upadacitinib;
- said antipsychotic agents are selected from haloperidol, fluphenazine, trifluoperazine, perphenazine, loxapine, chlorpromazine, thioridazine, risperidone, olanzapine, quetiapine, ziprasidone, aripiprazole, brexipiprazole, clozapine, lurasidone, asenapine, and cariprazine; and
- said anticonvulsive agents are selected from sodium channel blockers, calcium channel blockers, gamma-amino butyric acid (GABA) enhancers, glutamate inhibitors, and mixed/multiple mechanism anticonvulsive agents; wherein said sodium channel blockers are, preferably, selected from phenytoin, carbamazeoine, lamotrigine, and oxcarbazepine; said calcium channel blockers are, preferably, selected from ethosuximide, gabapentin, and pregabalin; said gamma-amino butyric acid (GABA) enhancers are, preferably, selected from phenobarbital, clonazepam, diazepam and vigabatrin; said glutamate inhibitors are, preferably, selected from topiramate and felbamate; and said mixed/multiple mechanism anticonvulsive agents are, preferably, selected from valproate, levetiracetam, zonisamide, rufinamide, lacosamiode, perampanel, and brivaracetam.

In one embodiment, the mucoadhesive layer does not comprise any of the following:
crosslinked polyacrylic acid, copolymers of methyl vinyl ether and maleic anhydride, poly(methacrylates), polyvinyl caprolactam-polyvinyl acetate-polyethylene glycol graft copolymers, and polyoxyl castor oils.

In one embodiment, said one or several pharmaceutically acceptable excipient(s) is(are) selected from plasticizers, pH-adjusting agents, chelating agents, viscosity modifiers, film-forming agents, solubilizers, taste-masking agents, API-stabilizing agents, release-modifying agents and colorants.

In one embodiment, the mucoadhesive layer comprises the following wt.% proportions:
- 30 - 97 wt.% of said non-crosslinked linear polyacrylic acid;
- 3 - 40 wt.% of said active pharmaceutical ingredient (API); and
- optionally, 0.01 - 60 wt.% of said one or several pharmaceutically acceptable excipient(s);

wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In a preferred embodiment, the mucoadhesive layer comprises the following wt.% proportions:
a) 64 - 97 wt.% of said non-crosslinked linear polyacrylic acid;
   - 3 - 36 wt.% of said active pharmaceutical ingredient (API);
   optionally, 0.01 - 10 wt.% of said one or several pharmaceutically acceptable excipient(s); or
b) 30 - 60 wt.% of said non-crosslinked linear polyacrylic acid;
   3 - 35 wt.% of said active pharmaceutical ingredient (API); and
   30 - 60 wt.% of said one or several pharmaceutically acceptable excipient(s);
   Wherein, in both a) and b), wt.% is with reference to the total dry weight of said mucoadhesive layer;
   with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In a further preferred embodiment, the mucoadhesive layer comprises the following wt.% proportions:
a) 65 - 91 wt.%, preferably 68 - 85 wt.%, of said non-crosslinked linear polyacrylic acid;
   9 - 35 wt.% , preferably 15 - 32 wt.%, of said active pharmaceutical ingredient (API);
   optionally, 0.01 - 10 wt.% of said one or several pharmaceutically acceptable excipient(s) ; or
b) 32 - 55 wt.% of said non-cross-linked linear polyacrylic acid;
   5 - 25 wt.% of said active pharmaceutical ingredient (API); and
   32 - 55 wt.% of said one or several pharmaceutically acceptable excipient(s);
   wherein, in both a) and b), wt.% is with reference to the total dry weight of said mucoadhesive layer;
   with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In yet a further preferred embodiment, the mucoadhesive layer comprises the following wt.% proportions:
a) 94 - 97 wt.%, 92 - 93 wt.%, 89 - 91 wt.%, 87 - 88 wt.%, 84 - 86 wt.%, 82 - 83 wt.%, 79 - 81 wt.%, 77 - 78 wt.%, 74 - 76 wt.%, 72 - 73 wt.%, 69 - 71 wt.%, 67 - 68 wt.%, or 64 - 66 wt.% of said non-crosslinked linear polyacrylic acid; 3 - 6 wt.%, 7 - 8 wt.%, 9 - 11 wt.%, 12 - 13 wt.%, 14 - 16 wt.%, 17 - 18 wt.%, 19 - 21 wt.%, 22 - 23 wt.%, 24 - 26 wt.%, 27 - 28 wt.%, 29 - 31 wt.%, 32 - 33 wt.%, or 34 - 36 wt.% of said active pharmaceutical ingredient (API);
   optionally, 0.01 - 10 wt.% of said one or several pharmaceutically acceptable excipient(s); or
b) 33 - 54 wt.% of said non-cross-linked linear polyacrylic acid;
   10 - 20 wt.% of said active pharmaceutical ingredient (API);
   33 - 54 wt.% of said one or several pharmaceutically acceptable excipient(s);
   wherein, in both a) and b), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In one embodiment, the mucoadhesive layer comprises the following weight proportions:
a) 82 - 84 wt.% of said non-cross-linked linear polyacrylic acid;
   15 - 18 wt.% of said active pharmaceutical ingredient (API);
   optionally, 0.01 - 3 wt.% of said one or several pharmaceutically acceptable excipient(s); or
b) 67 - 69 wt.% of said non-cross-linked linear polyacrylic acid;
   30 - 33 wt.% of said active pharmaceutical ingredient (API);
   optionally, 0.01 - 3 wt.% of said one or several pharmaceutically acceptable excipient(s); or
c) 52 - 54 wt.% of said non-cross-linked linear polyacrylic acid;
   11 - 12 wt.% of said active pharmaceutical ingredient (API);
   33 - 36 wt.% of said one or several pharmaceutically acceptable excipient(s); or
d) 33 - 36 wt.% of said non-cross-linked linear polyacrylic acid;
   11 - 13 wt.% of said active pharmaceutical ingredient (API);
   52 - 55 wt.% of said one or several pharmaceutically acceptable excipient(s);

wherein, in a), b), c) and d), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In one embodiment, said one or several pharmaceutically acceptable excipient(s) is(are) selected from a combination of: a) hydroxpropyl cellulose (HPC), polyvinylpyrrolidone (PVP), a plasticizer, such as glycerol, a colorant, and, optionally, a taste-masking or sweetening agent; and b) vinylpyrrolidone-vinylacetate-copolymer (e.g. Kollidon^{®} VA64), a plasticizer, such as glycerol, a colorant, and, optionally, a taste-masking or sweetening agent.

In one embodiment, said one or several pharmaceutically acceptable excipient(s) is(are) a combination of hydroxypropyl cellulose (HPC), polyvinylpyrrolidone (PVP), a plasticizer, such as glycerol, a colorant, and, optionally, a taste-masking or sweetening agent; wherein said mucoadhesive layer comprises 12 - 13 wt.% hydroxypropyl cellulose (HPC), 11 - 13 wt.% polyvinylpyrrolidone, 4 -5 wt.% plasticizer, 5 - 6 wt.% colorant and, optionally, 0.01 - 0.2 wt.% of a taste-masking or sweetening agent;
wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In one embodiment, said one or several pharmaceutically acceptable excipient(s) is(are) a combination of vinylpyrrolidone-vinylacetate-copolymer (e.g. Kollidon^{®}), a plasticizer, such as glycerol, a colorant, and, optionally, a taste-masking or sweetening agent; wherein said mucoadhesive layer comprises 42 - 45 wt.% vinylpyrrolidone-vinylacetate-copolymer (e.g. Kollidon^{®}), 4 - 5 wt.% plasticizer, 5 - 6 wt.% colorant and, optionally, 0.01- 0.2 wt.% of a taste-masking or sweetening agent;
wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

In one embodiment, said non-crosslinked linear polyacrylic acid has a molecular weight in the range of from 6kDa to 700 kDa, preferably from 100 kDa to 700 kDa, more preferably from 300 kDa to 700 kDa, even more preferably from 400 to 700 kDa, even more preferably from 500 to 700 kDa, even more preferably in the range of from 500 to 600 kDa.

In one embodiment, the mucoadhesive layer has a thickness in the range of from 20 µm to 1500 µm, preferably 20 µm to 1000 µm, more preferably 20 µm to 800 µm, more preferably 30 µm to 600 µm, even more preferably 40 µm to 400 µm, yet even more preferably 50 µm to 200 µm. and most preferably 60 µm to 150 µm; wherein said thickness refers to a thickness of said mucoadhesive layer in a dry state; alternatively said mucoadhesive layer having a thickness in the range of from 20 µm to 1500 µm, preferably 20 µm to 1000 µm, more preferably 20 µm to 800 µm, more preferably 30 µm to 600 µm, even more preferably 40 µm to 500 µm yet even more preferably 90 µm to 450 µm, yet even more preferably 90 µm to 420 µm; wherein said thickness refers to a thickness of said mucoadhesive layer in a dry state.

In one embodiment, said mucoadhesive layer comprises said active pharmaceutical ingredient (API) in an amount in the range of from 0.01 mg to 10 mg per cm² of mucoadhesive layer, or in an amount of 0.05 - 200 mg per g of mucoadhesive layer.

In one embodiment, said active pharmaceutical ingredient (API) is an immunosuppressant, wherein, preferably, said immunosuppressant is selected from tacrolimus, sirolimus, mycophenolic acid and its prodrugs.

In a further aspect, the present invention also relates to a mucoadhesive film, preferably a mucoadhesive buccal film, comprising a mucoadhesive layer according to the present invention as defined herein, and further comprises a backing layer attached to said mucoadhesive layer, wherein said backing layer, preferably, is permeable for water.

In one embodiment of the mucoadhesive film according to the present invention, said backing layer comprises a polymer selected from cellulose, cellulose derivatives, including hydroxypropyl cellulose (HPC), hydroxyethyl cellulose (HEC), hydroxypropylmethyl cellulose (HPMC), ethyl cellulose (EC), propyl cellulose (PC), and alginates, and wherein furthermore, said backing layer optionally comprises a plasticizer.

In one embodiment, said backing layer has a time-dependent dissolution in water or aqueous solutions, with a defined first dissolution time, and wherein said mucoadhesive layer also has a time-dependent dissolution in water or aqueous solutions with a defined second dissolution time, wherein said first dissolution time is smaller than, equal to or larger than said second dissolution time. ; wherein, preferably, said first dissolution time is in the range of from 1 min to 60 min, more preferably, in the range of from 1 min to 45 min, even more preferably in the range of from 1 min to 30 min.

In yet a further aspect, the present invention also relates to a method of preparing a mucoadhesive layer, as defined herein, said method comprising the steps:
a) Providing, in any order but separate from each other, an active pharmaceutical ingredient (API), a non-crosslinked linear polyacrylic acid, and, optionally, one or several pharmaceutically acceptable excipient(s), said API, said non-crosslinked linear polyacrylic acid and said one or several pharmaceutically acceptable excipient(s) being as defined herein;
b) dissolving said active pharmaceutical ingredient (API), said non-crosslinked linear polyacrylic acid, and, optionally, said one or several pharmaceutically acceptable excipient(s) in a suitable organic or aqueous solvent, preferably selected from ethanol, acetone, isopropanol, dichloromethane (DCM), water and mixtures of any of the foregoing, thereby obtaining a viscous solution;
c) casting said viscous solution as a layer on a plane surface;
d) drying said casted layer, preferably at a temperature in the range of from 20 °C to 60 °C, more preferably 20 °C to 50 °C, wherein, optionally, said drying is performed under vacuum conditions;
e) releasing said dried layer from said plane surface, thereby obtaining a mucoadhesive layer as defined herein.

In yet a further aspect, the present invention also relates to a method of preparing a mucoadhesive film, as defined herein, said method comprising the steps:
a') preparing, in any order, a mucoadhesive layer and a backing layer, wherein preparing said mucoadhesive layer is performed in accordance with the preceding aspect and/or described herein;
b') laminating said backing layer to said mucoadhesive layer, under suitable conditions of temperature and/or pressure, such that said backing layer becomes attached to said mucoadhesive layer, and such that no air bubbles get trapped between said mucoadhesive layer and said backing layer.

### DETAILED DESCRIPTION OF THE INVENTION

The present inventors have surprisingly found that a mucoadhesive layer comprising non-crosslinked linear polyacrylic acid, an active pharmaceutical ingredient (API), and optionally one or several pharmaceutically acceptable excipient(s), allows for an efficient incorporation of such API into the layer, and advantageously enables a subsequent release of the API from the layer, when and where such release is intended. Such layer provides for a high, stable drug loading and significantly improves drug release profiles in comparison to other buccal administration forms. Moreover, the non-crosslinked linear polyacrylic acid forms a polymer matrix into which the API is incorporated, which polymer matrix acts as a reservoir for such API. Without wishing to be bound by any theory, the present inventors believe that the respective API is incorporated in the polymer matrix in dissolved, suspended or emulsified form, preferably in a dissolved form, such that such API can be considered to be in a "solid solution"-form. Because the API released from the mucoadhesive layer according to the present invention is absorbed directly into the blood stream through the mucous membranes, the inventive mucoadhesive layer is particularly suitable for such APIs which otherwise, i. e. when orally administered, would be subject to a first-pass effect and/or would be metabolized in the gastrointestinal tract. That is, mucoadhesive layer(s) according to the present invention are particularly suitable for such APIs which, upon oral administration to a patient, are subject to an intestinal metabolization by a patient's intestinal bacteria and/or are subject to an intestinal metabolization by a patient's own enzyme systems that are present in the patient's intestinal cells, e. g. cytochrome-P 450-enzyme(s) (CYP).

The mucoadhesive layer(s) according to the present invention is(are) also particularly suitable for APIs which are substrates of the P-glycoprotein-efflux transporter system, also called ATP-binding cassette sub-family B member 1 ABCB1 or multidrug resistance protein 1 MDR1, or APIs which are resorbed very poorly for other reasons, when administered to the gastrointestinal tract, for example by oral administration.

The term "polyacrylic acid" as used herein, refers to a homopolymer of acrylic acid. The term "homopolymer", as used herein, refers to a polymer in which at least 90 mol% of the units in the polymer are derived from acrylic acid, or at least 95 mol% or at least 98 mol%, or 100 mol% of the units in the polymer are derived from acrylic acid. The term "linear", when used in connection with "polyacrylic acid" is meant to refer to a polyacrylic acid which has substantially no-crosslinking. To make this sufficiently clear, sometimes herein also the term "non-crosslinked linear" is used, and the two terms "linear" and "non-crosslinked linear" are used interchangeably or together herein. These terms refer to polymers in which crosslinking (or branching) occurs, on average, at fewer than one in ten of the polyacrylic acid-units in the longest chain of the polymer, or at fewer than one in twenty, or one in fifty, of the polyacrylic acid-units in the longest chain of the polymer. The cross-linked density can also be defined as the inverse of the molecular weight between cross-links (Me); and may be no more than 0.0014, or no more than 0.0007. Non-crosslinked linear polyacrylic acid can be obtained commercially from various sources, for example in various types, with different molecular weights, from Lubrizol Corporation.

The term "polyacrylic acid" as used herein, is meant to encompass both the protonated and deprotonated forms of such acid, i. e. wherein the respective acid groups are in their respective acid-form, i. e. -COOH-form, and their respective carboxylate-form, i. e. -COO⁻-form. Both forms are meant to be encompassed by the term "polyacrylic acid", as used herein.

In preferred embodiments, the non-crosslinked linear polyacrylic acid, as used in accordance with the present invention has a molecular weight in the range of from 6 kDa to 700 kDa, preferably from 100 kDa to 700 kDa, more preferably from 300 kDa to 700 kDa, even more preferably from 400 to 700 kDa, even more preferably from 500 to 700 kDA, even more preferably in the range of from 500 to 600 kDa. As used herein, the molecular weight, preferably is a weight average molecular weight (M_{w}) which is determined by size exclusion chromatography (SEC), preferably, as follows:
A liquid sample is prepared of about 1.5 g/L (0.15%) polymer in 0. 1M NaNOs at pH 10. The sample is filtered, prior to injection. 100 pL of the filtered sample is injected into the column (TOSOH Bioscience, 2x TSKgel PWxL columns plus TSKgel Guard) using 0.1M NaNOs in deionized water at pH 10 as the mobile phase. The flow rate is 0.7 mL/min. A Viscotek Triple Detector Array (TDA) (Malvern Panalytical) is used as the detector. This detector incorporates RI, Light Scattering, and Viscosity detectors. The instrument is calibrated with a single narrow MW standard of polyethylene oxide (PEO). A commercially-available sample of polyacrylic acid is used as a linear reference polymer. Mobile phase (and sample) enters the TDA and passes through the GPC/SEC chromatography columns. The columns are maintained at the same temperature as the detectors (40 °C). After eluting from the column, the dissolved polymer molecules, now separated by size, pass through the three detectors. Finally, the mobile phase passes through the viscometer before going to waste.

The RI detector gives information about the concentration of the components in the sample. The light scattering detectors respond to the intensity of light scattered by the sample which is related to molecular weight and also allows the Rg of large molecules to be calculated. The viscometer measures the changing solution viscosity to calculate the intrinsic viscosity of the sample (not used for viscosity determination herein).

In one embodiment, the non-crosslinked linear polyacrylic acid has a Brookfield viscosity of at least 100 cP (cP = mPa.s), or at least 200 cP, or at least 250 cP, or at least 300 cP, or at least 400 cP.

As used herein, Brookfield viscosity is measured using a Brookfield viscometer model DV2TRV, 20 rpm, at 25 °C on an aqueous solution containing 4 wt. % of the PAA at pH 7.5. The spindles used with this model are RV01 - RV07 with the following viscosity range covered: RV-01, up to 500 cP; RV-02, up to 2000 cP; RV-03, up to 5000 cP; RV-04, up to 10,000 cP; RV-05, up to 20,000 cP: RV-06, up to 50,000 cP, and RV-07, up to 200,000 cP. The aqueous solution is formed by dissolving the PAA in water and adjusting the pH to 7.5 using an 18% aqueous solution of NaOH.

Brookfield viscosity correlates well with molecular weight. Brookfield viscosity is proportional to molecular weight, as determined by the described methods. For example, a linear PAA polymer having a Brookfield viscosity of 200 cP has an Mₙ of 162,048 Da and an M_{w} of 545,692 Da; and a linear PAA polymer having a Brookfield viscosity of 2075 cP has an Mₙ of 527,772 Da and an M_{w} 1,071,000 Da.

In accordance with the present invention, the mucoadhesive layer shows an excellent API release behavior as for example measured in a so-called "transwell assay", in which a mucoadhesive layer, in accordance with the present invention or a mucoadhesive film, in accordance with the present invention, i. e. a mucoadhesive layer with a laminated backing layer, is placed on an agarose gel, prepared using an aqueous solution of agarose at a concentration of 20 mg/ml. The agarose is soaked in acceptor medium (10% (w/w) methyl-beta-cyclodextrin in buffer solution); the assembly of film in contact with the agarose gel is incubated at 37°C under shaking (400 rpm) for defined period of time, and at defined intervals, samples from the acceptor medium are taken and measured for API-content. Release is expressed as µg release per film area or as "% (µg released / µg applied)" allowing comparison of different film formulations in terms of in vitro release properties.

In accordance with the present invention, the mucoadhesive layer/film has a release characteristic of at least 20% (w/w) API, as measured in the aforementioned transwell assay, preferably, at least 40% (w/w) API, more preferably, at least 60% (w/w) API. The in vitro testing as described above allows a rating of formulations. This in vitro rating as exemplified in Example 2, could also be confirmed in the in vivo study described in Example 3.

In accordance with embodiments of the present invention, a mucoadhesive layer also comprises one or several pharmaceutically acceptable excipient(s). Preferably, such pharmaceutically acceptable excipient(s) is (are) selected from plasticizers, pH adjusting agents, chelating agents, taste-masking agents, API-stabilizing agents, release-modifying agents and colorants.

Examples of suitable plasticizers in accordance with embodiments of the present invention are glycerol, polyethylene glycol, propylene glycol, triethyl citrate, diethyl phthalate, sorbitol, and acetyl tributyl citrate. These and other plasticizers help in enhancing the mechanical properties of the mucoadhesive layer/film according to the present invention.

Examples of suitable pH-adjusting agents are citric acid, sodium bicarbonate, sodium carbonate, sodium acetate, hydrochloric acid, sodium hydroxide, lactic acid, potassium hydroxide, acetic acid, tris(hydroxymethyl)aminomethane and phosphoric acid. These pH-adjusting agents help in maintaining the stability, solubility, and bioavailability of the API.

Examples of suitable chelating agents in accordance with the present invention are EDTA, disodium EDTA, citric acid, trisodium citrate, tartaric acid, sodium tartrate, sodium glycinate, dimercaprol deferoxamine, and phytic acid. These chelating agents help in stabilizing the formulation by binding to metal ions that could otherwise catalyze the degradation or negatively interfere with the API.

Examples of suitable viscosity modifiers in accordance with the present invention are hydroxypropyl cellulose (e.g. Klucel GF ^{®}), carboxymethylcellulose, hydroxyethylcellulose, methylcellulose, polyvinyl alcohol (PVA), xanthan gum, carbomers (e.g. Carbopol ^{®}), guar gum and pectin. In a preferred embodiment, said viscosity modifier is hydroxypropyl cellulose.

Examples of suitable film-forming agents in accordance with the present invention are vinylpyrrolidone-vinylacetate-copolymers (e.g. Kollidon ^{®}, in particular Kollidon ^{®} VA64), polyvinylpyrrolidone (PVP), polyvinyl alcohol (PVA), xanthan gum, polyethylene oxide, and gelatin. In a preferred embodiment, said film-forming agent is a vinylpyrrolidone-vinylacetate-copolymer.

Examples of suitable solubilizers in accordance with the present invention are D-α-Tocopherol-Polyethylenglycol-Succinate (TPGS), polysorbates, polyethylene glycols (PEG), propylene glycol, polyethoxylated castor oils (e.g. Cremophor/Kolliphor ^{®}), glycerol, sorbitan esters, cyclodextrins, and lecithin. In a preferred embodiment, said solubilizer is D-α-Tocopherol-Polyethylenglycol-Succinat (TPGS).

Examples of suitable taste-masking agents in accordance with the present invention are sucralose, aspartame, mannitol, sodium saccharin, xylitol, erythritol, flavoring agents, and cyclodextrins. These taste-masking agents help to improve the palatability of the mucoadhesive layer(s)/film(s) in accordance with the present invention, ensuring better patient compliance and adherence to the prescribed treatment.

Examples of API-stabilizing agents, in accordance with the present invention, are antioxidants, preservatives, buffering agents, chelating agents, surfactants, and cryoprotectants; examples for antioxidants are e. g. ascorbic acid, examples of preservatives are methylparaben; examples of buffering agents are sodium phosphate or potassium phosphate, examples of chelating agents are EDTA and citric acid, examples of surfactants are polysorbate 80 (Tween80), and sodium lauryl sulfate (SDS); examples of cryoprotectants are trehalose and mannitol.

Examples of release-modifying agents in accordance with the present invention are polyvinyl alcohol, methacrylic acid copolymers, sodium alginate, xanthan gum, polyethylene oxide and gelatine. These release-modifying agents help to ensure a sustained, controlled or delayed release formulation.

Examples of colorants in accordance with the present invention are titanium dioxide, talcum, brilliant blue, alura red, sunset yellow, iron oxides, tartrazine, carmine, beta-carotene and the like. Such colorants may be used to improve the appearance of a layer/film in accordance with the present invention, facilitating the identification thereof and enhancing patient compliance.

Moreover, reference is made to the figure(s), wherein
**Figure 1** shows the results of an in-vivo study performed on mini-pigs using various examples of mucoadhesive buccal films in accordance with the present invention, in comparison to other films.
**Figure 2** shows the results of an in-vivo study performed on Sprague Dawley rats using an example mucoadhesive buccal film in accordance with the present invention, in comparison to other films and oral administration of the API (in this case tacrolimus).
**Figure 3** shows the results of a further in-vivo study performed on Sprague Dawley rats using example mucoadhesive buccal films in accordance with the present invention.

Furthermore, in the following, reference is made to the examples, which are given to illustrate, not to limit the present invention.

### EXAMPLES

### Example 1 - Film preparation

### 1.1 General description

This protocol is used for the preparation of a bi-layered mucoadhesive buccal film by casting. The backing layer is casted separately and laminated onto the active to release the active on buccal application directly into the mucosa minimizing its release into the oral cavity. The active layer can be blue colored using brilliant blue as coloring agent to allow easy discrimination of active and backing layer. The casting mass is prepared by dissolving all components in ethanol. Ensure that all equipment is appropriately prepared.

### 1.2 Film preparation

The film preparation includes the preparation of the mucoadhesive active layer, the supporting backing layer and the final lamination.

### 1.2.1 Active Layer

### Preparation of the film mass

Non-crosslinked linear polyacrylic acid was obtained commercially as "Apinovex^{®}" from Lubrizol Corporation. The exemplary type used in the present exemplary studies was "Apinovex^{®} LV", which is a linear non-crosslinked polyacrylic acid of a molecular weight of 540 - 550 kDa and/or a viscosity in the range of from 200 - 220 cP.

The respective amount of Apinovex^{®} LV polymer is weighed into a 100 mL amber screw-top jar. The Apinovex polymer is then dissolved using the respective solvent with or without the addition of brilliant blue. The respective amount of the API is then added to the solution. The mixture is stirred for a minimum of 4 hours at 600 rpm using a magnetic stirrer. Subsequently, the mixture is agitated for additional 2 hours at 350 rpm on an orbital shaker. The process results in a clear, dark blue, and viscous mass which is ready for film casting.

### Film casting and drying

Film casting was carried out similarly for all tested compositions. Drying of the film masses was either carried out at room temperature overnight or at elevated temperatures. Table 1 summarizes the general film preparation parameters. The applied gap size, drying temperature and drying times of the respective batches can be found in the respective table.

**Table 1. Film casting parameters and equipment.**

| | |
|---|---|
| **Casting liner / material** | Loparex siliconized foil 75µm |
| **Casting instrument** | Byko-drive S (Byk) |
| **Casting equipment** | film casting knife, adjustable (ByK) |
| **Casting width** | 10 cm |
| **Casting speed** | 20 mm/s |

### Cutting

The dried films were carefully removed from the siliconized foil using tweezers, placed on a glass plate and cut into appropriate film pieces using a scalpel and a template with rectangles measuring 2.5 cm × 2.0 cm.

### 1.2.2 Backing Layer

### Preparation of the film mass

The film mass preparation of the backing layer was performed as followed. 13.3 g of Protanal 10/60, 7.8 g of D-sorbitol, 6.6 g of glycerol, 213.3 g of demineralized water and 0.6 g of Bayferrox Yellow were added to the mixing cup of a vacuum blender. The mixture was homogenized for 40 minutes under vacuum. Between each run, the mixture was allowed to cool to room temperature for 5-10 minutes. Then the mixture was decanted into four 50 ml Falcons and centrifuged at 4000 rpm for 10 minutes.

### Film casting and drying

After cooling to room temperature, the film mass was casted and dried using the parameters illustrated in Table 2.

**Table 2. Film preparation parameters of backing layer.**

| | |
|---|---|
| **Casting liner / material** | Glass |
| **Casting instrument** | Byko-drive S (Byk) |
| **Casting equipment** | film casting knife, adjustable |
| **Casting width** | 10 cm |
| **Casting speed** | 20 mm/s |
| **Casting Gap/height** | 2000 µm |
| **Drying temperature** | 50 °C |
| **Drying Time** | 2 hours |

### Cutting

The dried films were carefully removed from the glass substrate using tweezers, placed on a glass plate and cut into appropriate film pieces using a scalpel and a template with rectangles measuring 2.5 cm × 2.0 cm.

### 1.2.3 Lamination

The lamination of the backing layer and the active layer was performed as described in the following. The backing layer was carefully wetted with demineralized water in the area where the active layers belonged. Using tweezers, the active Layer was carefully placed on the backing layer and centered. The active layer was carefully pressed on until no air bubbles were visible between the active and backing layer. The complete film consisting of the two layers were detached from the glass plate and placed on a plastic lid. The films were then dried for 2 hours at room temperature.

### 1.3 Characterization

The film masses were evaluated using light microscope with a 10-fold and 60-fold magnification.

Characterization of dried film was performed as followed:
- Area weight: A film cut-out with a dimension of 5 - 10 cm² was weighed and the area weight was calculated in g/m².
- Thickness: The film was measured with the layer thickness gauge Trotec BB20 at 10 measurement points. The film was layered with an object glass, which was measured at three points in advance.
- Residual moisture: A film cut-out with a dimension of 10 cm² was weighed, dried at 50 °C for 24 h and weighed again. The mass difference represents the residual moisture and was calculated in %.
- Light microscope: Images of the film were taken with 10-fold and 6o-fold magnification.
- In vitro permeation: The permeation was evaluated using a transwell assay setup ("Transwell Release"). Such assay is herein also sometimes referred to as a "transwell assay". Briefly, an agarose gel with a thickness of 5-10 mm was placed on a permeable polycarbonate membrane (Corning, 734-1567). A 10-25 % Methyl-β-cyclodextrine solution as used as acceptor medium. A 8 mm diameter round film cutout was placed on the agarose gel at t=0 and sealed to prevent water evaporation. The sample was shaken at 37°C and 400 rpm. The film was removed after 2 hours.

Samples were collected from the acceptor media after several time intervals (100 µL). The API concentration was determined either using HPLC or UV-VIS spectroscopy. The higher the concentration in these samples, the higher the release of API from the film. Such release is expressed as "% (w/w)", and refers to the percentage of API that has been released from the film, with reference to the total amount of API originally incorporated in the film.

### Example 2: Composition of Apinovex-based buccal films products

The compositions of the films and the characterization thereof were as follows:

### Tacrolimus

| **Description** | | **RC-P-079** | **RC-P-090** | **RC-P-091** | **RC-P-097** | **RC-P-098** | **RC-P-109** | **RC-P-123** | **RC-P-123_01** | **RC-P-124** | **RC-P-126** | **RC-P-126_01** | **RC-P-127_01** |
|---|---|---|---|---|---|---|---|---|---|---|---|---|---|
| **Zusammensetzung** | | | | | | | | | | | | | |
| Tacrolimus | g | 1,201 | 1,209 | 2,803 | 2,803 | 2,803 | 0,756 | 1,502 | 1,502 | 3,505 | 5,022 | 5,022 | 1,517 |
| Apinovex | g | 6,033 | 6,051 | 5,999 | 5,999 | 5,999 | 3,752 | 7,500 | 7,500 | 7,506 | 15,067 | 15 067 | 14,989 |
| Ethanol | g | 33,81 | 32,81 | 32,95 | 32,95 | 32,95 | 20,23 | 41,03 | 41,03 | 39,04 | 80,39 | 80,39 | 83,62 |
| TPGS | g | | | | | | 0,26 | | | | | | |
| Glycerol | g | | | | | 0,33 | | | | | | | |
| Brilliant blue | g | 0,0034 | 0,0033 | 0,0033 | 0,0033 | 0,0033 | 0,0020 | 0,0041 | 0,0041 | 0,0039 | 0,0080 | 0,0080 | 0,0084 |
| **total weight** | **g** | **41,0** | **40,1** | **41,8** | **41,8** | **42,1** | **25,0** | **50,0** | **50,0** | **50,1** | **100,5** | **100,5** | **100,1** |
| **total weight solids** | **g** | **7,24** | **7,26** | **8,80** | **8,80** | **9,14** | **4,77** | **9,01** | **9,01** | **11,01** | **20,10** | **20,10** | **16,51** |

| **Film Cast liquid** | | | | | | | | | | | | | |
|---|---|---|---|---|---|---|---|---|---|---|---|---|---|
| Tacrolimus | % | 2,92 | 3,02 | 6,71 | 6,71 | 6,66 | 3,03 | 3,00 | 3,00 | 7,00 | 5,00 | 5,00 | 1,51 |
| Apinovex | % | 14,70 | 15,10 | 14,37 | 14,37 | 14,25 | 15,01 | 14,99 | 14,99 | 14,99 | 14,99 | 14,99 | 14,97 |
| Ethanol | % | 82,37 | 81,88 | 78,91 | 78,91 | 78,29 | 80,93 | 82,00 | 82,00 | 78,00 | 80,00 | 80,00 | 83,51 |
| TPGS | % | 0,00 | 0,00 | 0,00 | 0,00 | 0,00 | 1,03 | 0,00 | 0,00 | 0,00 | 0,00 | 0,00 | 0,00 |
| Glycerol | % | 0,00 | 0,00 | 0,00 | 0,00 | 0,79 | 0,00 | 0,00 | 0,00 | 0,00 | 0,00 | 0,00 | 0,00 |
| Brilliant blue | % | 0,008 | 0,008 | 0,008 | 0,008 | 0,008 | 0,008 | 0,008 | 0,008 | 0,008 | 0,008 | 0,008 | 0,008 |
| **SUM total** | % | **100,0** | **100,0** | **100,0** | **100,0** | **100,0** | **100,0** | **100,0** | **100,0** | **100,0** | **100,0** | **100,0** | **100,0** |
| **SUM solids** | % | **17,6** | **18,1** | **21,1** | **21,1** | **21,7** | **19,1** | **18,0** | **18,0** | **22,0** | **20,0** | **20,0** | **16,5** |

| **Film solid** | | | | | | | | | | | | | |
|---|---|---|---|---|---|---|---|---|---|---|---|---|---|
| Tacrolimus | % | 16,6 | 16,6 | 31,8 | 31,8 | 30,7 | 15,9 | 16,7 | 16,7 | 31,8 | 25,0 | 25,0 | 9,2 |
| Apinovex | % | 83,4 | 83,3 | 68,1 | 68,1 | 65,6 | 78,7 | 83,3 | 83,3 | 68,1 | 75,0 | 75,0 | 90,8 |
| TPGS | % | 0,0 | 0,0 | 0,0 | 0,0 | 0,0 | 5,4 | 0,0 | 0,0 | 0,0 | 0,0 | 0,0 | 0,0 |
| Glycerol | % | 0,0 | 0,0 | 0,0 | 0,0 | 3,6 | 0,0 | 0,0 | 0,0 | 0,0 | 0,0 | 0,0 | 0,0 |
| Brilliant blue | % | 0,05 | 0,05 | 0,04 | 0,04 | 0,04 | 0,04 | 0,05 | 0,05 | 0,04 | 0,04 | 0,04 | 0,05 |
| **SUM** | % | **100,0** | **100,0** | **100,0** | **100,0** | **100,0** | **100,0** | **100,0** | **100,0** | **100,0** | **100,0** | **100,0** | **100,0** |

| **Casting Parameters** | | | | | | | | | | | | | |
|---|---|---|---|---|---|---|---|---|---|---|---|---|---|
| Gap size | µm | 1000 | 1000 | 1000 | 1000 | 1000 | 1000 | 1000 | 1000 | 1000 | 1000 | 1000 | 1000 |
| Drying Temperature | °C | RT | RT | RT | RT | RT | 30-60 | RT | RT | RT | RT | RT | RT |
| Drying time | h | over night | over night | over night | over night | over night | 1,25 | over night | over night | over night | over night | over night | over night |

| **Characterization** | | | | | | | | | | | | | |
|---|---|---|---|---|---|---|---|---|---|---|---|---|---|
| Area weight | g/m² | 116 | 198 | 158 | 151 | 174 | 126 | 134 | 173 | 178 | 160 | 138 | 200 |
| Thickness | µm | 90 | | 125 | 129 | 130 | n/a | 123 | 128 | 114 | n/a | n/a | n/a |
| Disintegration Time | min:sec | 00:54 | 01:56 | 04:37 | 02:47 | 01:46 | n/a | 01:13 | 01:54 | 01:53 | n/a | n/a | n/a |
| Residual moisture | % | 2.9 | 7,9 | 7,0 | 5,9 | 6.2 | 16,9 | 4,2 | 8,9 | 8,1 | 10,8 | 12,2 | 12,4 |
| TWA Release (8h) | % | 62 | 34 | 49 | 16 | 17 | 46 | 42 | 47 | 11 | 24 | 17 | 36 |

The release of API in the transwell assay ("TWA Release") was in the range of from 11% to 62%.

### Rapamycin

| **Description** | | **RC-P_133** | **RC-P_135** | **RC-P_136** | **RC-P_137** | **RC-P_138** | **RC-P_141** | **RC-P-150** | **RC-P-151** | **RC-P-164_1** | **RC-P-167_1** | **RC-P-170_1** |
|---|---|---|---|---|---|---|---|---|---|---|---|---|
| **Composition** | | | | | | | | | | | | |
| Rapamycin | g | 0,297 | 1,529 | 0,302 | 0,901 | 1,500 | 3,507 | 2,101 | 1,500 | 0,150 | 0,302 | 0,604 |
| Apinovex | g | 4,500 | 4,501 | 4,547 | 4,513 | 4,543 | 7,546 | 4,506 | 4,502 | 4,521 | 4,503 | 4,562 |
| Ethanol | g | 25,02 | 24,03 | 25,20 | 24,60 | 24,00 | 39,02 | 23,41 | 24,02 | 25,12 | 24,76 | 24,47 |
| Glycerol | g | | | | | | | | | 0,46 | 0,45 | 0,48 |
| Brilliant blue | g | 0,0011 | 0,0011 | 0,0011 | 0,0011 | 0,0011 | 0,0011 | | | 0,0025 | 0,0025 | 0,0025 |
| **total weight** | **g** | **29,8** | **30,1** | **30,1** | **30,0** | **30,0** | **50,1** | **30,0** | **30,0** | **30,3** | **30,0** | **30,1** |
| **total weight solids** | **g** | **4,80** | **6,03** | **4,85** | **5,42** | **6,04** | **11,05** | **6,61** | **6,00** | **5,13** | **5,26** | **5,65** |

| **Film Cast liquid** | | | | | | | | | | | | |
|---|---|---|---|---|---|---|---|---|---|---|---|---|
| Rapamycin | % | 1,00 | 5,08 | 1,00 | 3,00 | 4,99 | 7,01 | 7,00 | 5,00 | 0,50 | 1,01 | 2,00 |
| Apinovex | % | 15,09 | 14,97 | 15,13 | 15,03 | 15,12 | 15,07 | 15,01 | 15,00 | 14,94 | 15,00 | 15,15 |
| Ethanol | % | 83,91 | 79,94 | 83,86 | 81,96 | 79,88 | 77,92 | 77,99 | 80,01 | 83,05 | 82,48 | 81,25 |
| Glycerol | % | 0,00 | 0,00 | 0,00 | 0,00 | 0,00 | 0,00 | 0,00 | 0,00 | 1,51 | 1,50 | 1,59 |
| Brilliant blue | % | 0,004 | 0,004 | 0,004 | 0,004 | 0,004 | 0,002 | 0,000 | 0,000 | 0,008 | 0,008 | 0,008 |
| **SUM total** | % | **100,0** | **100,0** | **100,0** | **100,0** | **100,0** | **100,0** | **100,0** | **100,0** | **100,0** | **100,0** | **100,0** |
| **SUM solids** | % | **16,1** | **20,1** | **16,1** | **18,0** | **20,1** | **22,1** | **22,0** | **20,0** | **17,0** | **17,5** | **18,8** |

| **Film solid** | | | | | | | | | | | | |
|---|---|---|---|---|---|---|---|---|---|---|---|---|
| Rapamycin | % | 6,2 | 25,3 | 6,2 | 16,6 | 24,8 | 31,7 | 31,8 | 25,0 | 2,9 | 5,7 | 10,7 |
| Apinovex | % | 93,8 | 74,6 | 93,8 | 83,3 | 75,2 | 68,3 | 68,2 | 75,0 | 88,1 | 85,6 | 80,8 |
| Glycerol | % | 0,0 | 0,0 | 0,0 | 0,0 | 0,0 | 0,0 | 0,0 | 0,0 | 8,9 | 8,6 | 8,5 |
| Brilliant blue | % | 0,02 | 0,02 | 0,02 | 0,02 | 0,02 | 0,01 | 0,00 | 0,00 | 0,05 | 0,05 | 0,04 |
| **SUM** | % | **100,0** | **100,0** | **100,0** | **100,0** | **100,0** | **100,0** | **100,0** | **100,0** | **100,0** | **100,0** | **100,0** |

| **Casting Parameters** | | | | | | | | | | | | |
|---|---|---|---|---|---|---|---|---|---|---|---|---|
| Gap size | µm | 1000 | 1000 | 1000 | 1000 | 1000 | 1000 | 1000 | 1000 | 1000 | 1000 | 1000 |
| Drying Temperature | °C | RT | RT | RT | RT | RT | RT | RT | RT | 50 | 50 | 50 |
| Drying time | h | over night | over night | over night | over night | over night | over night | over night | over night | 1 h | 1 h | 1 h |

| **Characterization** | | | | | | | | | | | | |
|---|---|---|---|---|---|---|---|---|---|---|---|---|
| Area weight | g/m² | 109 | 132 | 107 | 105 | 119 | 75 | 140 | 125 | 152 | 168 | 192 |
| Thickness | µm | 119 | 114 | 103 | 119 | 153 | 129 | 134 | 110 | 129 | 131 | 139 |
| Disintegration Time | min:sec | 00:18 | 02:36 | 00:33 | 00:15 | 02:27 | 01:32 | 07:51 | 04:42 | 01:06 | 01:10 | 01:18 |
| Residual moisture | % | 12,7 | 12,8 | 11,4 | 11,3 | 11,3 | 12,8 | 8,8 | 9,3 | 4,9 | 5,0 | 5,2 |
| TWA Release (8h) | % | n/a | n/a | 66 | 31 | 15 | 10 | 11 | 10 | 60 | 47 | 41 |

The release of API in the transwell assay ("TWA Release") was in the range of from 10% to 66%.

### Itraconazole

| **Description** | | **RC-P_140** | **RC-P-152** |
|---|---|---|---|
| **Composition** | | | |
| Itraconazole | g | 0,630 | 0,196 |
| Apinovex | g | 2,495 | 4,501 |
| Ethanol | g | 12,36 | 12,65 |
| Dichlormethane | | 12,36 | 12,65 |
| Brilliant blue | g | | 0,0011 |
| **total weight** | **g** | **27,9** | **30,0** |
| **total weight solids** | **g** | **3,12** | **4,70** |

| **Film Cast liquid** | | | |
|---|---|---|---|
| Itraconazole | % | 2,26 | 0,65 |
| Apinovex | % | 8,96 | 15,00 |
| Ethanol | % | 44,39 | 42,17 |
| Dichlormethane | % | 44,39 | 42,17 |
| Brilliant blue | % | 0,000 | 0,004 |
| **SUM total** | % | **100,0** | **100,0** |
| **SUM solids** | % | **11,2** | **15,7** |

| **Film solid** | | | |
|---|---|---|---|
| Itraconazole | % | 20,2 | 4,2 |
| Apinovex | % | 79,8 | 95,8 |
| Brilliant blue | % | 0,00 | 0,02 |
| **SUM** | % | **100,0** | **100,0** |

| **Casting Parameters** | | | |
|---|---|---|---|
| Gap size | µm | 1000 | 1000 |
| Drying Temperature | °C | RT | RT |
| Drying time | h | over night | over night |

| **Characterization** | | | |
|---|---|---|---|
| Area weight | g/m² | 133 | 100 |
| Thickness | µm | 92 | 211 |
| Disintegration Time | min:sec | 08:53 | 01:01 |
| Residual moisture | % | 5,7 | 10,3 |
| TWA Release (8h) | % | 76 | 105 |

The release of API in the transwell assay ("TWA Release") was in the range of from 76% to 100%.

### Prednisolon

| **Description** | | **RC-P-147** | **RC-P-148** | **RC-P-149** | **RC-P-154** | **RC-P-154_01** | **RC-P-155** | **RC-P-155_01** |
|---|---|---|---|---|---|---|---|---|
| **Composition** | | | | | | | | |
| Prednisolon | g | 0,987 | 0,600 | 0,205 | 0,192 | 0,192 | 0,603 | 0,603 |
| Apinovex | g | 3,001 | 3,001 | 3,005 | 4,531 | 4,531 | 4,501 | 4,501 |
| Acetone | g | 13,02 | 13,20 | 13,35 | 12,47 | 12,47 | 12,54 | 12,54 |
| Ethanol | g | 13,02 | 13,20 | 13,35 | 12,47 | 12,47 | 12,54 | 12,54 |
| Brilliant blue | g | | 0,0011 | 0,0011 | 0,0011 | 0,0011 | 0,0011 | 0,0011 |
| **total weight** | **g** | **30,0** | **30,0** | **29,9** | **29,7** | **29,7** | **30,2** | **30,2** |
| **total weight solids** | **g** | **3.99** | **3,60** | **3,21** | **4,72** | **4,72** | **5,11** | **5,11** |

| **Film Cast liquid** | | | | | | | | |
|---|---|---|---|---|---|---|---|---|
| Prednisolon | % | 3,29 | 2,00 | 0,68 | 0,65 | 0,65 | 2,00 | 2,00 |
| Apinovex | % | 9,99 | 10,00 | 10,05 | 15,28 | 15,28 | 14,91 | 14,91 |
| Acetone | % | 43,36 | 44,00 | 44,63 | 42,04 | 42,04 | 41,54 | 41,54 |
| Ethanol | % | 43,36 | 44,00 | 44,63 | 42,04 | 42,04 | 41,54 | 41,54 |
| Brilliant blue | % | 0,000 | 0,004 | 0,004 | 0,004 | 0,004 | 0,004 | 0,004 |
| **SUMME total** | % | **100,0** | **100,0** | **100,0** | **100,0** | **100,0** | **100,0** | **100,0** |
| **SUMME solids** | % | **13.3** | **12,0** | **10,7** | **15,9** | **15,9** | **16,9** | **16,9** |

| **Film solid** | | | | | | | | |
|---|---|---|---|---|---|---|---|---|
| Prednisolon | % | 24,8 | 16,7 | 6,4 | 4,1 | 4,1 | 11,8 | 11,8 |
| Apinovex | % | 75,2 | 83,3 | 93,6 | 95,9 | 95,9 | 88,2 | 88,2 |
| Brilliant blue | % | 0,00 | 0,03 | 0,03 | 0,02 | 0,02 | 0,02 | 0,02 |
| **SUM** | % | **100,0** | **100,0** | **100,0** | **100,0** | **100,0** | **100,0** | **100,0** |

| **Casting Paramete** | | | | | | | | |
|---|---|---|---|---|---|---|---|---|
| Gap size | µm | 1000 | 2000 | 2000 | 1000 | 1500 | 1000 | 1500 |
| Drying Temperature | °C | RT | RT | RT | RT | RT | RT | RT |
| Drying time | h | over night | over night | over night | over night | over night | over night | over night |

| **Characterization** | | | | | | | | |
|---|---|---|---|---|---|---|---|---|
| Area weight | g/m² | 133 | 124 | 111 | 124 | 160 | 116 | 127 |
| Thickness | µm | 92 | 152 | 94 | 78 | 117 | 94 | 165 |
| Disintegration Time | min:sec | 08:53 | 01:04 | 00:41 | 00:37 | 01:13 | 00:39 | n/a |
| Residual moisture TWA Release (8h) | % | 5,7 | 6,3 | 6,9 | 11,8 | 12,4 | 11,6 | 11,3 |
| | % | 70 | 73 | 88 | 103 | 96 | 71 | n/a |

The release of API in the transwell assay ("TWA Release") was in the range of from 70% to 100%.

Overall, different API classes can be embedded in the non-linear polyacrylic acid matrix. All the films show an extremely good, concentration-dependent release profile as measured by an in vitro transwell assay as described herein.

### Example 3: Animal studies

### a) Studies involving mini pigs

Various mucoadhesive films involving tacrolimus ("TAC") as API were produced in accordance with the procedures described in Example 1, and their respective composition is indicated hereafter in the following table. It should be noted that the films designated as "Tac-MBF2" and "Tac-MBF6" are exemplary embodiments in accordance with the present invention, whereas the other films are cellulose-based ("Tac-OTF2") or Carbopol-based ("Tac-MBF1_1") and "Tac-MBF4, which are not in accordance with the present invention and are for reference.

The compositions of these films were as follows:

### Composition of films

| **Name** | Tac-OTF 2 | Tac-MBF 1_1 | Tac-MBF 4 | Tac-MBF 2 | Tac-MBF 6 |
|---|---|---|---|---|---|
| **Batch No.** | | 20240131_1 | 20240213 | 20240207 | 20240219_15/7 |

| | | | | | |
|---|---|---|---|---|---|
| **size of film (cm²)** | 5 | 5 | 5 | 5 | 5 |
| **nominal dose of film (mg)** | 14,2 | 6,4 | 6,6 | 8,9 | 19,8 |
| **Assay** | 98,5% | 90,1 % | 93,3% | 85,1% | 86,7% |

| **Formuation No.** | | | | | |
|---|---|---|---|---|---|
| **mucoadhesive layer (w/w)** | | RC-P-076 | RC-P-085 | RC-P-079 | RC-P-091 |
| **TAC** | 19,23 | 11,10 | 13,63 | 16,86 | 31,80 |
| **HPC Klucel EF** | 41,73 | | | | |
| **HPC Klucel GF** | 15,00 | | | | |
| **EC N50NF** | 5,00 | | | | |
| **Polycarbophil** | 2,00 | | | | |
| **Kollidon SR** | 5,00 | | | | |
| **Carbopol 971P NF** | | 74,03 | 68,15 | | |
| **Apinovex** | | | | 83,10 | 68,20 |
| **Transcutol** | | 14,81 | | | |
| **Eudragit L100-55** | | | 18,17 | | |
| **Glycerol** | 4,77 | | | | |
| **Nippon talk** | 5,19 | | | | |
| **Iron oxide yellow** | 1,73 | | | | |
| **Brilliant Blau** | | 0,06 | 0,05 | 0,05 | 0,05 |
| **Tartaric acid** | 0,25 | | | | |
| **Neotam** | 0,10 | 1 | | | |
| **SUM** | 100 | 100 | 100 | 100 | 100 |
| **Area weight / g/m²** | 150 | 142 | 122 | 124 | 146 |

| **backing layer (w/w)** | | RC-W-048 | RC-W-048 | RC-W-048 | RC-W-048 |
|---|---|---|---|---|---|
| **HEC** | 25,95 | | | | |
| **HPC EF** | 29,45 | | | | |
| **HPC GF** | 12,85 | | | | |
| **Kollidon SR** | 29,45 | | | | |
| **Protanal 10/60** | | 46,84 | 46,84 | 46,84 | 46,82 |
| **Glycerol** | | 23,45 | 23,45 | 23,45 | 23,4 |
| **Sorbitol** | | 27,59 | 27,59 | 27,59 | 27,59 |
| **Iron oxide yellow** | 2 | 2,12 | 2,12 | 2,12 | 2,19 |
| **Brilliant blue** | 0,2 | | | | |
| **Neotam** | 0,1 | | | | |
| **SUM** | 100 | 100 | 100 | 100 | 100 |
| **Area weight** / **g/m²** | 80 | 178 | 178 | 178 | 166 |

The aforementioned films were used for animal studies involving minipigs. All animals were 5 to 6 months old at the time of treatment with the body weight between 11 and 14 kg. Animals were fasting (over night) for at least 16 hours before treatment and anesthetized during treatment. The film was placed with the mucoadhesive layer site on the mucosa in the animal's mouth and likely pressed until the film adhered. The film remained on the buccal mucosa for 60 minutes and was then removed. Blood samples were collected via a venous catheter. For determination of blood levels of tacrolimus (TAC), 2 ml of blood was collected from all animals into K2ETDA-code tubes. Samples were collected at the following time points: Immediately before administration (0 min), then at 1, 1.5, 2, 2.5, 3, 4, 6, 8, 12, 18 and 24hours after administration of the film. After blood collection, the blood tubes were cooled on ice and stored in a freezer (at a temperature in the range of -30°C to -15°C) until analysis. The concentration of tacrolimus was determined by reliable LC-MS/MS method.

Pharmacokinetic parameters were calculated from the time course of blood concentration. The calculated AUC was normalized with the active dose and set in relation to the individual body weight of the animal.

The results of the mean pharmacokinetic parameters are shown in the following table:

### Mean pharmacokinetic parameters of films (mini pig, n=5)

| **Name** | Tac-OTF 2 | Tac-MBF 2 | Tac-MBF 6 | Tac-MBF 1_1 | Tac-MBF 4 |
|---|---|---|---|---|---|
| **Type** | Cellulose | Apinovex | Apinovex | Carbopol & Transcutol | Carbopol & L100-55 |
| **Dose per film** / **mg** | 14,2 | 8,9 | 19,8 | 6,4 | 6,6 |
| **Body weight / kg** | 12,48 | 14,56 | 13,98 | 12,84 | 13,98 |
| **dose/BW** | 1,140 | 0,612 | 1,418 | 0,501 | 0,474 |
| **AUC_24h** | 22,305 | 41,129 | 117,872 | 2,902 | 4,344 |
| **AUC_24h/Dose** | 1,573 | 4,625 | 5,953 | 0,453 | 0,658 |
| **AUC_24h/dose/BW** | 19,185 | 66,887 | 82,457 | 5,733 | 9,103 |

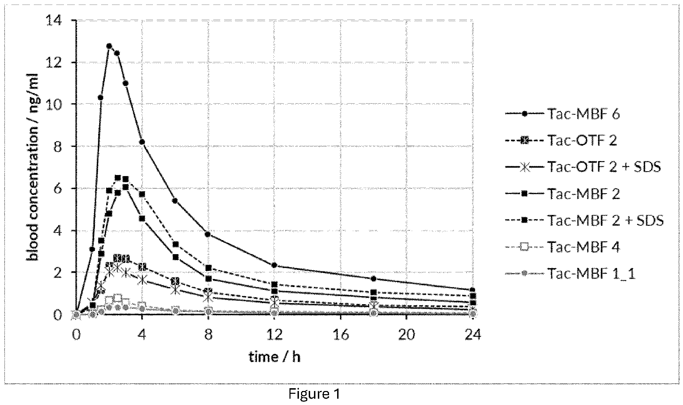

The results are also shown in Figure 1 from which it becomes clear that the mean blood concentration of tacrolimus achieved is by far the highest in the two example films in accordance with the present invention ("Tac-MBF6" and "Tac-MBF2", with, and without SDS), whereas only lower blood levels are achieved in the reference films.

The results show that the mucoadhesive layer-film in accordance with the present invention shows an excellent release profile of the API, in comparison to the other films tested.

### b) Studies involving rats - Part I

### Apinovex-based mucoadhesive buccal films (Study No. 2023NCM005)

The tested films were flexible, flat circular films consisting of different layers. The mucoadhesive layer contained the drug substance Tacrolimus. The layer containing Tacrolimus adheres to the mucosal surface and is intended to be placed sublingual under the tongue of the animal. The backing layer does not contain drug substance and minimizes drug release into the oral cavity, maximizing transmucosal diffusion. It is yellow colored. The mucoadhesive layer are designed to deliver the drug within approximately 60 min; during that time the mucoadhesive layer might erode to certain degree while the backing layer is not dissolving.

Film dimensions are 0.28 cm² area and 6 mm diameter of the mucoadhesive layer.

### Composition of film

| | |
|---|---|
| **Batch No.** | 20230831 |
| **Date of manufacturing** | 31.08.2023 |

| | |
|---|---|
| **size of film (cm²)** | 0.28 |
| **nominal dose of film (mg)** | 0.58 |

| **Formulation No.** | |
|---|---|
| **mucoadhesive layer (w/w)** | 2308_15/3 |
| **TAC** | 17.6 |
| **Apinovex** | 82.4 |
| **SUM** | 100 |
| **Area weight / g/m²** | 113 |

| **backing layer (w/w)** | BL55 |
|---|---|
| **Protanal 10/60** | 48.4 |
| **Glycerol** | 26.2 |
| **Sorbitol** | 23.2 |
| **Iron oxide yellow** | 2.2 |
| **SUM** | 100 |
| **Area weight** / **g/m²** | 213 |

### In-vivo experiment

Five adult male Sprague Dawley rats were purchased from Janvier Labs (France) The animals were housed in a separate temperature-controlled room (20-24°C) and maintained in a 12h light/12h dark cycle. Food and water were available *ad libitum* throughout the duration of the study. Body weight of each individual animal was recorded at the day of treatment.

At the day of treatment, the individual body weight of the animals was recorded.

Each animal was treated once. Animals were anesthetized by intraperitoneal injection prior to the application of the test item. The oral cavity was moistened with 10 µL saline solution and the film was placed under the tongue at time point t = 0 of each animal which were placed into the cage until recovery from anesthesia with the drug-containing mucoadhesive layer in contact with the mucosa. The backing layer of the films was then moistened with 10 µL of saline. The film was removed after the residence time of 20 min. Recovery from anesthesia took place around 45 minutes after introduction.

Blood samples were collected by puncture of the lateral tail vein at the following time points: predose, 30 min, 45 min, 1 h, 2 h, 4 h, 8 h and 24 h post dose.

At each of the designated time points 20 µl blood was collected from the tail vein into K3-EDTA capillaries (Minivette POCT, SARSTEDT). The blood samples were transferred into polypropylene tubes and frozen on dry ice within 1-2 min upon sampling and stored at -20°C until LC-MS analysis for quantification of Tacrolimus in whole blood.

Pharmacokinetic analysis of mean curves was performed.

The dose normalized systemic exposure was calculated: AUCo-tz values were divided by the corresponding dose expressed as mg/kg body weight to eliminate the body weight differences.

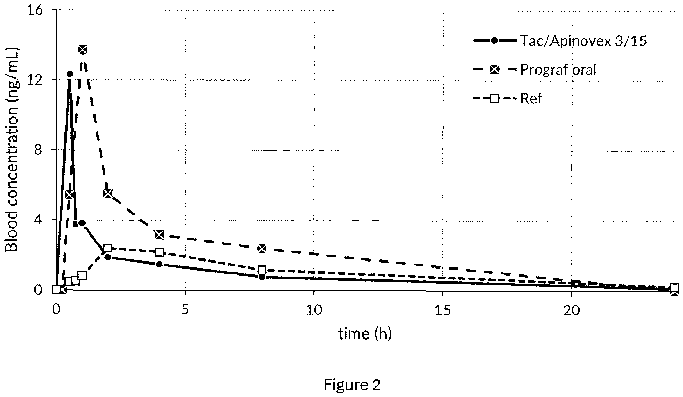

Results are shown in Figure 2 and the following table:

### Mean pharmacokinetic profile (rats, n=5, normalized to 0.58 mg)

| **Study No.** | **2023NCM005** | **2024NCM002** | **2023FLM004** |
|---|---|---|---|
| **Day of treatment** | 04.09.2023 | 14.03.2024 | 17.05.2023 |
| **Parameter** | | | |
| **Formulation type** | **TAC/Apinovex** | **Solid Solution** | **Prograf** |
| Formulation ID | **2308_15/3** | **Tac-OTF** 2 | |
| Batch no. | **20230831** | | **5E3107D** |
| Dose (mg) | 0.58 | 1.60 | 0.50 |
| Area (cm²) | 0.28 | 0.56 | n.a. |
| Residence time (min) | 20 | 20 | n.a. |
| Application route | sublingual | sublingual | oral |

| **Mean PK parameters** | | | |
|---|---|---|---|
| Number of animals | 5 | 10 | 5 |
| tmax (h) | 0.5 | 2.0 | 1 |
| Body weight (g) | 435 | 626 | 371 |
| **AUCo-tz/dose/body weight** (*) **(ng*h/ml** / **mg/kg)** | **18** | **13** | **22** |

| | | | |
|---|---|---|---|
| (*) = normalized to equal film area | | | |

From the data, it can be concluded that the Apinovex-based film formulation significantly better performed in vivo in terms of achieved mean AUC levels as compared to the competitor cellulose-based prototype. Data also suggest that the Apinovex-film tested in this setting (sublingual, 20 minutes application time) is much more competitive to the bioavailability of the reference product Prograf and this formulation approach could be further developed to a marketable product.

### c) Studies involving rats - Part II

### Apinovex-based mucoadhesive buccal films (Study No. 2024NCM003)

The tested films were flexible, flat circular films consisting of different layers. The mucoadhesive layer contained the drug substance Tacrolimus. The layer containing Tacrolimus adheres to the mucosal surface and is intended to be placed sublingual under the tongue of the animal. The backing layer does not contain drug substance and minimizes drug release into the oral cavity, maximizing transmucosal diffusion. The mucoadhesive layer is designed to deliver the drug within approximately 60 min; during that time the mucoadhesive layer might erode to a certain degree whilst the backing layer is also dissolving.

Film dimensions are 0.28 cm² area and 6 mm diameter.

### Composition of films

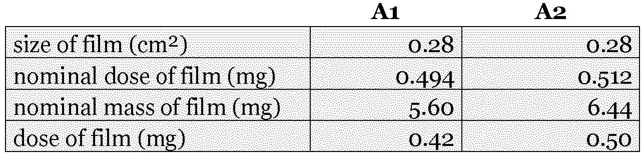

| **Composition of mucoadhesive layer (w/w)** | | |
|---|---|---|
| Tacrolimus | 11.76 | 12.20 |
| Apinovex | 53.76 | 34.13 |
| HPC Klucel GF | 12.42 | |
| PVP Kgo | 12.00 | |
| Kollidon VA64 | | 43.46 |
| Glycerol | 4.77 | 4.84 |
| Nippon talk | 5.19 | 5.27 |
| Neotam | 0.10 | 0.10 |
| SUM | 100 | 100 |

| **Composition backing layer (w/w)** | | |
|---|---|---|
| HEC | 25 | 25 |
| HPC EF | 29 | 29 |
| HPC GF | 12 | 12 |
| Kollidon SR | 31.7 | 31.7 |
| Iron oxide yellow | 2 | 2 |
| Brilliant blue | 0.2 | 0.2 |
| Neotam | 0.1 | 0.1 |
| SUM | 100 | 100 |

### In-vivo experiment

Twenty adult male Sprague Dawley rats were purchased from Janvier Labs (France). The animals were housed in a separate temperature-controlled room (20-24°C) and maintained in a 12h light/12h dark cycle. Food and water were available *ad libitum* throughout the duration of the study. Body weight of each individual animal was recorded at the day of treatment.

At the day of treatment, the individual body weight of the animals was recorded.

Each animal was treated once. Animals were anesthetized by intraperitoneal injection prior to the application of the test item. The oral cavity was moistened with 10 µL saline solution and two films were placed under the tongue at time point t = o of each animal which were placed into the cage until recovery from anesthesia with the drug-containing mucoadhesive layer in contact with the mucosa. The backing layer of the films was then moistened with 10 µL of saline. The film was removed after a residence time of 20 min. Recovery from anesthesia took place around 45 minutes after introduction.

Blood samples were collected by puncture of the lateral tail vein at the following time points: predose, 30 min, 45 min, 1 h, 2 h, 4 h and 8 h post dose.

At each of the designated time points 20 µl blood was collected from the tail vein into K3-EDTA capillaries (Minivette POCT, SARSTEDT). The blood samples were transferred into polypropylene tubes and frozen on dry ice within 1-2 min upon sampling and stored at -20°C until LC-MS analysis for quantification of Tacrolimus in whole blood.

### Mean pharmacokinetic profile (rats, n=io per formulation)

### Pharmacokinetic analysis was performed.

The dose normalized systemic exposure was calculated: AUCo-tz values were divided by the corresponding dose expressed as mg/kg body weight to eliminate the body weight differences.

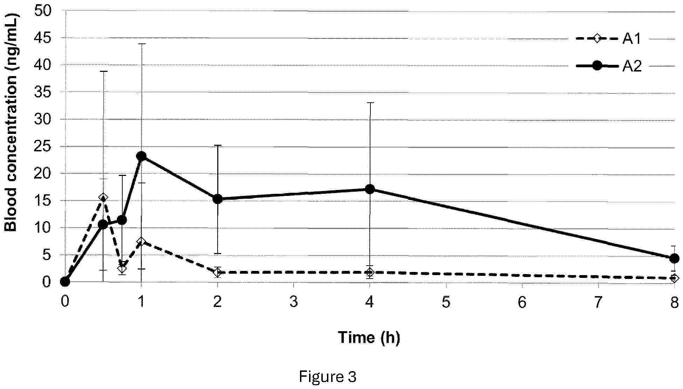

Results are shown in Figure 3 and the following table:

### Summary of pharmacokinetic parameters

| | | **A1** | **A2** |
|---|---|---|---|
| AUCextr | % | 33.2 | 29.4 |
| AUClast | h*ng/ml | 19.3 | 95.5 |
| AUClast/Dose | h*kg*ng/ml/mg | 5.51 | 27.3 |
| Cmax | ng/ml | 21.6 | 30.4 |
| Cmax/Dose | kg*ng/ml/mg | 6.17 | 8.67 |
| tmax | h | 1.03 | 2.0 |

From the data, it can be concluded that inclusion of further excipients, e.g. plasticizers, viscosity enhancers, viscosity modifiers, matrix formers, film-forming agents, solubilizers, taste-masking agents, sweeteners, colorants, etc., in order to optimize film properties and manufacturability still results in considerable blood levels of the used API Tacrolimus.

## Claims

1. A mucoadhesive layer for buccal administration of an active pharmaceutical ingredient (API), said layer comprising:
- non-crosslinked linear polyacrylic acid;
- an active pharmaceutical ingredient (API),
- optionally, one or several pharmaceutically acceptable excipient(s).

2. The mucoadhesive layer for buccal administration according to claim 1, wherein said active pharmaceutical ingredient is selected from immunosuppressants, antifungal agents, neurotransmitters, antiasthmatic agents, anti-obesity drugs, nutraceuticals, elastase inhibitors, analgesics, anthelmintics, anti-arrhythmic agents, antibiotics, anticoagulants, antidepressants, antidiabetics, antihistamines, antihypertensive agents, antimuscarinic agents, antimycobacterial agents, antineoplastic agents, antiemetics, antithyroid agents, antiviral agents, anxiolytic sedatives, astringents, beta-adrenoreceptor blocking agents, cardiovascular agents, cardiac inotropic agents, contrast agents, corticosteroids, cough suppressants, diagnostic imaging agents, diuretics, dopaminergics, haemostatics, lipid regulating agents, muscle relaxants, parasympathomimetics, bisphosphonates, prostaglandins, radiopharmaceuticals, hormones, therapeutic peptides, anti-allergic agents, stimulants, anorectics, sympathomimetics, vasodilatators, antirheumatic agents, antipsychotic agents, and anticonvulsive agents;

3. The mucoadhesive layer according to any of claims 1 - 2, wherein the immunosuppressant is selected from macrolides, corticosteroids, anti-metabolites, non-macrolide calcineurin inhibitors, and other immunosuppressant drugs,
wherein, preferably,
- said macrolides are selected from tacrolimus, sirolimus, everolimus and pimecrolimus;
- said corticosteroids are selected from cortisone, prednisone, hydrocortisone, prednisolone, methylprednisolone, dexamethasone, betamethasone, beclomethasone, alclometasone, flumetasone, fluticasone, mometasone, budesonide, ciclesonide, and desonide;
- said antimetabolites are selected from methotrexate, azathioprine, mercaptopurine, and fluorouracil;
- said non-macrolide calcineurin inhibitors are selected from ciclosporin and voclosporin; and
- said other immunosuppressant drugs are selected from fingolimod, TNF-α-binding proteins, such as infliximab, etanercept and adalimumab; mycophenolic acid and its prodrugs, such as mycophenolate mofetil and mycophenolate sodium; and fingolimod.

4. The mucoadhesive layer according to any of the foregoing claims, wherein
- said antifungal agents are selected from itraconazole, ketoconazole, fluconazole, clotrimazole, miconazole, econazole, tioconazole, posaconazole and voriconazole;
- said neurotransmitters are selected from dopamine, levodopa, carbidopa, adrenaline (ephinephrin), noradrenalin (norepinephrin), acetylcholine, serotonine, glutamate, glycine, gamma-aminobutyric acid (GABA), oxytocin, endorphins, enkephalins, and histamines;
- said antiasthmatic agents are selected from beta-2-agonists, corticosteroids, leukotriene modifiers, mast cell stabilisers, methylxanthines, anticholinergics. biologic agents, and combinations for inhalation...;
- said anti-obesity drugs are selected from appetite suppressants. Lipase inhibitors, GLP-1 receptor agonists, serotonin 2C agonists, and sympathomimetic amines;
- said nutraceuticals are selected from vitamins, minerals, omega-3 fatty acids, omega-6 fatty acids, herbal supplements, probiotics, prebiotics, amino acid supplements, protein supplements, antioxidants, fibre supplements, botanical extracts, and specialty nutrients;
- said elastase inhibitors are selected from alpha-1 antitrypsin (AAT), sivelestat, elafin, AZD9668, ONO-5046, serpina1, peptide-based elastase inhibitors, small molecule elastase inhibitors, epigallocatechin gallate (EGCG), and diosmin;
- said analgesics are selected from non-opioid analgesics, nonsteroidal anti-inflammatory drugs (NSAIDs), opioid analgesics, adjuvant analgesics, topical analgesics, and combination analgesics;
- said anthelmintics are selected from benzimidazoles, avermectins, pyrantel pamoate, praziquantel, diethyl carbamazine, niclosamide, levamisole, piperazine, oxamniquine, and bithionol;
- said anti-arrhythmic agents are selected from sodium channel blockers, beta blockers, potassium channel blockers, calcium channel blockers, and other anti-arrhythmic agents not falling under any of the aforementioned blockers and being selected from adenosine, digoxine, and magnesium sulfate;
- said antibiotics are selected from beta-lactams, macrolides, quinolones, fluoroquinolones, aminoglycosides, tetracyclines, sulfonamides, glycopeptides, oxazolidinones, lincosamides, nitroimidazoles, polypeptide antibiotics, rifamycins, and other antibiotics not falling under any of the aforementioned antibiotic classes and being selected from chloramphenicol, daptomycin, fosfomycin, and nitrofurantoin;
- said anticoagulants are selected from vitamin K antagonists, direct oral anticoagulants, heparins, synthetic pentasaccharides, parenteral direct thrombin inhibitors, coumarins, and other anticoagulants not falling under any of the aforementioned anticoagulant classes and being selected from danaparoid and desirudin;
- said antidepressants are selected from selective serotonin reuptake inhibitors (SSRIs), serotonin-norepinephrine reuptake inhibitors (SNRIs), tricyclic antidepressants (TCAs), monoamine oxidase inhibitors, atypical antidepressants, serotonin modulators, noradrenergic and specific serotonergic antidepressants (NaSSAs), and serotonin antagonist and reuptake inhibitors;
- said antidiabetics are selected from insulins, biguanides, sulfonylureasmeglitinides, thiazolidinediones (TZDs), dipeptidyl peptidase-4 (DPP-4) inhibitors, glucagon-like peptide-1 (GLP-1) receptor agonists, sodium-glucose co-transporter-2 (SGLT2) inhibitors, alpha-glucosidase inhibitors, amylin analogues, bile acid sequestrants, and dopamine agonists;
- said antihistamines are selected from Diphenhydramine, Chlorpheniramine (Chlor-Trimeton), Brompheniramine, Clemastine, Hydroxyzine, Promethazine, Doxylamine, Meclizine, Dimenhydrinate, Loratadine, Cetirizine, Fexofenadine, Desloratadine, Levocetirizine, Bilastine, Rupatadine, Olopatadine, Azelastine, and Ketotifen;
- said antihypertensive agents are selected from diuretics, beta blockers, angiotensin-converting enzyme (ACE) inhibitors, angiotensin II receptor blockers (ARBs), calcium chzannel blockers (CCBs), alpha blockers, centrally acting antihypertensive agents, direct vasodilators, and renin inhibitors;
- said antimuscarinic agents are selected from Oxybutynin, Tolterodine, Solifenacin, Darifenacin, Trospium, Fesoterodine, Ipratropium, Tiotropium, Aclidinium, Umeclidinium, Hyoscyamine, Dicyclomine, Glycopyrrolate, Scopolamine, Benztropine, Trihexyphenidyl, Atropine, Tropicamide, Cyclopentolate;
- said antimycobacterial agents are selected from Isoniazid, Rifampin, Ethambutol, Pyrazinamide, Rifabutin, Rifapentine, Streptomycin, Amikacin, Kanamycin, Capreomycin, Ethionamide, Prothionamide, Cycloserine, Para-aminosalicylic acid (PAS), Levofloxacin, Moxifloxacin, Ciprofloxacin, Bedaquiline, Delamanid, Linezolid, Dapsone, Rifampin, Clofazimine, Clarithromycin, Azithromycin, Amikacin, Ethambutol, Rifabutin, Rifampin, Ciprofloxacin, Levofloxacin, Moxifloxacin, Linezolid
- said antineoplastic agents are selected from alkylating agents, antimetabolites, natural product antineoplastic agents, antitumor antibiotics, hormonal antineoplastic agents, monoclonal antibodies, tyrosine kinase inhibitors, checkpoint inhibitors, platinum compounds, proteasome inhibitors, and immunomodulatory agents;
- said antiemetics are selected from serotonin (5-HT3) receptor antagonists, dopamine antagonists, NK1 receptor antagonists, antihistamines, anticholinergics, benzodiazepines, cannabinoids, and steroids;
- said antithyroid agents are selected from thionamides, iodine solution, and radioactive iodine;
- said antiviral agents are selected from nucleoside and nucleotide analogues, protease inhibitors, non-nucleoside reverse transcriptase inhibitors (NNRTIs), integrase inhibitors, fusion inhibitors, neuraminidase inhibitors, polymerase inhibitors, entry inhibitors, small molecule antiviral agents not falling under any of the aforementioned antiviral agent classes;
- said anxiolytic sedatives are selected from benzodiazepines, non-benzodiazepine anxiolytics, barbiturates, antihistamines, beta blockers, selective serotonin reuptake inhibitors (SSRIs), serotonin-norepinephrine reuptake inhibitors (SNRIs), and tricyclic antidepressants;
- said astringents are selected from astringent aluminium compounds, witch hazel extract, tannins, calamine, astringent zinc compounds, astringent silver compounds, astringent alcohols, astringent acids, and astringent herbal agents;
- said beta-adrenoreceptor blocking agents are selected from non-selective beta-blockers, selective beta-blockers, beta-blockers with intrinsic sympathomimetic activity (ISA), and beta-blockers with alpha-blocking activity;
- said cardiovascular agents are selected from antihypertensive agents, antiarrhythmic agents, anticoagulants, antiplatelet agents, lipid-lowering agents, vasodilators, cardiac glycosides, inotropes, angiotensin receptor-neprilysin inhibitors (ARNIs), and aldosterone antagonists;
- said cardiac inotropic agents are selected from cardiac glycosides, sympathomimetic amines, phosphodiesterase inhibitors, calcium sensitizers, and beta-adrenergic agonists;
- said contrast agents are selected from iodinated contrast agents, gadolinium-based contrast agents, barium sulfate, microbubble contrast agents, and iron oxide nanoparticles;
- said corticosteroids are selected from glucocorticoids and mineralocorticoids;
- said cough suppressants are selected from opioid derivatives, non-opioid cough suppressants, and peripheral acting antitussive agents;
- said diagnostic imaging agents are selected from iodinated contrast media, gadolinium-based contrast agents, barium sulfate, microbubble contrast agents, radiopharmaceuticals, and fluorescent dyes;
- said diuretics are selected from thiazide diuretics, loop diuretics, potassium-sparing diuretics, carbonic anhydrase inhibitors, and osmotic diuretics;
- said dopaminergics are selected from dopamine precursors, dopamine agonists, monoamine oxidase-B (MAO-B) inhibitors, catechol-O-methyltransferase (COMT) inhibitors, and dopamine releasing agents;
- said hemostatics are selected from systemic hemostatics selected from antifibrinolytics, tranexamic acid, aminocaproic acid, vitamin K, phytonadione, desmopressin; topical hemostatics selected from gelatin sponges, oxidized cellulose, microfibrillar collagen, thrombin, fibrin, chitosan; and hemostatic dressing and agents selected from kaolin and zeolite;
- said lipid regulating agents are selected from statins, fibrates, bile acid sequestrants, niacin, cholesterol absorption inhibitors, omega-3 fatty acid ethyl esters, PCSK9 inhibitors and ATP citrate lyase (ACL) inhibitors;
- said muscle relaxants are selected from baclofen, cyclobenzaprine, methocarbamol, tizanidine, diazepam, carisoprodol, metaxalone, orphenadrine, and chlorzoxazone;
- said parasympathomimetics are selected from bethanechol, pilocarpine, carbachol, methacholine, cevimeline, neostigmine, pyridostigmine, physostigmine, edrophonium and rivastigmine;
- said bisphosphonates are selected from alendronate, risedronate, ibandronate, soledronate, etidronate and pamidronate;
- said prostaglandins are selected from prostaglandin E1 (PGE1, alprostadil), prostaglandin E2 (PGE2, dinoprostone), prostaglandin F2α (PGF2α, dinoprost), carboprost, misoprostol, latanoprost, travoprost, bimatoprost, and iloprost;
- said radiopharmaceuticals are selected from diagnostic and therapeutic radiopharmaceuticals, wherein said diagnostic radiopharmaceuticals are selected from Technetium-99m-based, Fluorine-18-based, Iodine-123-based, Gallium-67-based and Indium-111-based radiopharmaceuticals; and wherein said therapeutic radiopharmaceuticals are selected from Iodine-131-based, Yttrium -go-based, Samarium-153-based, Lutetium-177-based, and Radium-223-based radiopharmaceuticals;
- said hormones are selected from non-steroid hormones and steroid hormones; wherein said non-steroid hormones are selected from insulin, glucagon, thyroxine (T4), triiodothyronine (T3), parathyroid hormone (PTH), calcitonin, growth hormone (GH), prolactin, adrenocorticotropic hormone (ACTH), follicle-stimulating hormone (FSH) and luteinizing hormone; and wherein said steroid hormones are selected from cortisol, aldosterone, testosterone, estradiol, progesterone, dehydroepiandrosterone (DHEA), estrone, and estriol;
- said anti-allergic agents are selected from antihistamines, mast cell stabilizers, corticosteroids, leukotriene receptor antagonists, immunomodulators, and degongestants;
- said stimulants are selected from amphetamines, methylphenidates, xanthines, norepinephrine uptake inhibitors, modafinil-compounds, cocaine, ephedrine, and pseudoephedrine;
- said anorectics are selected from sympathomimetic anorectic compounds, serotonin-norepinephrine reuptake inhibitors (SNRIs), serotonin receptor agonists, GLP-1 receptor agonists, combination medications of stimulants and anticonvulsants or of antidepressants and opioid antagonists, and orlistat, wherein said combination medications are selected from the combinations phentermine/topiramate and bupropion/nalltroxone;
- said sympathomimetics are selected from direct-acting sympathomimetics, indirect-acting sympathomimetics, mixed-acting sympathomimetics, beta-agonists, and alpha-agonists, wherein said direct-acting sympathomimetics are selected from epinephrine, norepinephrine, dopamine, phenylephrine, dobutamine, and isoproterenol; said indirect-acting sympathomimetics are selected from amphetamine, methamphetamine, ephedrine and pseudoephedrine; saidf mixed-acting sympathomimetics are selected from ephedrine and metaraminol; said beta-agonists are selected from albuterol, salmeterol, formoterol and terbutaline; and said alpha-agonists are selected from clonidine, methyldopa and oxymetazoline;
- said vasodilatators are selected from nitrate compounds, calcium channel blockers, angiotensin-converting enzyme (ACE) inhibitors, angiotensin II receptor blockers (ARBs), direct vasodilators, phosphodiesterase inhibitors, potassium channel openers, endothelin receptor antagonists, and prostacyclin analogues; wherein said nitrate compounds are selected from nitroglycerin, isosorbide mononitrate, and isosorbide dinitrate; said calcium channel blockers are selected from amlodipine, nifedipine, verapamil, diltiazem; said angiotensin-converting enzyme (ACE) inhibitors are selected from enalapril, lisinopril, and ramipril; said angiotensin II receptor blockers (ARBs) are selected from losartan, valsartan, and candesartan; said direct vasodilators are selected from hydralazine and minoxidil; said phosphodiesterase inhibitors are selected from sildenafil, tadalafil, and milrinone; said potassium channel openers are selected from nicorandil and diazoxide; said endothelin receptor antagonists are selected from bosentan, and ambrisentan; and said prostacyclin-analogues are selected from epoprostenol, iloprost and treprostinil;
- said antirheumatic agents are selected from small molecule disease-modifying antirheumatic drugs (DMARDs), biologic disease-modifying antirheumatic drugs (DMARDs), and targeted synthetic disease-modifying antirheumatic drugs (DMARDs); wherein said small molecule disease-modifying antirheumatic drugs (DMARDs) are selected from methotrexate, sulfasalazine, hydroxychloroquine, leflunomide, azathioprine, cyclosporine, and cyclophosphamide; said biologic disease-modifying antirheumatic drugs (DMARDs) are selected from TNF-a inhibitors, B-cell inhibitors, T-cell co-stimulation modulators and interleukin inhibitors; and wherein said targeted synthetic disease-modifying antirheumatic drugs (DMARDs) are selected from janus kinase inhibitors;
- said antipsychotic agents are selected from haloperidol, fluphenazine, trifluoperazine, perphenazine, loxapine, chlorpromazine, thioridazine, risperidone, olanzapine, quetiapine, ziprasidone, aripiprazole, brexipiprazole, clozapine, lurasidone, ascenapine, and cariprazine; and
- said anticonvulsive agents are selected from sodium channel blockers, calcium channel blockers, gamma-amino butyric acid (GABA) enhancers, glutamate inhibitors, and mixed/multiple mechanism anticonvulsive agents; wherein said sodium channel blockers are selected from phenytoin, carbamazeoine, lamotrigine, and oxcarbazepine; said calcium channel blockers are selected from ethosuximide, gabapentin, and pregabalin; said gamma-amino butyric acid (GABA) enhancers are selected from phenobarbital, clonazepam, diazepam and vigabatrin; said glutamate inhibitors are selected from topiramate and felbamate; and said mixed/multiple mechanism anticonvulsive agents are selected from valproate, levetiracetam, zonisamide, rufinamide, lacosamiode, perampanel, and brivaracetam.

5. The mucoadhesive layer according to any of the foregoing claims, wherein the mucoadhesive layer does not comprise any of the following:
crosslinked polyacrylic acid, copolymers of methyl vinyl ether and maleic anhydride, poly(methacrylates), polyvinyl caprolactam-polyvinyl acetate-polyethylene glycol graft copolymers, and polyoxyl castor oils.

6. The mucoadhesive layer according to any of the foregoing claims, wherein said one or several pharmaceutically acceptable excipient(s) is(are) selected from plasticizers, pH-adjusting agents, chelating agents, viscosity modifiers, film-forming agents, solubilizers, taste-masking agents, API-stabilizing agents, release-modifying agents and colorants.

7. The mucoadhesive layer according to any of the foregoing claims, comprising the following wt.% proportions:
- 30 - 97 wt.% of said non-crosslinked linear polyacrylic acid;
- 3 - 40 wt.% of said active pharmaceutical ingredient (API); and
- optionally, 0.01 - 60 wt.% of said one or several pharmaceutically acceptable excipient(s);
wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

8. The mucoadhesive layer according to claim 7, comprising the following wt.% proportions:
a) 64 - 97 wt.% of said non-crosslinked linear polyacrylic acid;
- 3 - 36 wt.% of said active pharmaceutical ingredient (API);
optionally, 0.01 - 10 wt.% of said one or several pharmaceutically acceptable excipient(s); or
b) 30 - 60 wt.% of said non-crosslinked linear polyacrylic acid;
3 - 35 wt.% of said active pharmaceutical ingredient (API); and
30 - 60 wt.% of said one or several pharmaceutically acceptable excipient(s);
wherein, in both a) and b), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

9. The mucoadhesive layer according to claim 7 or 8, comprising the following wt.% proportions:
a) 65 - 91 wt.%, preferably 68 - 85 wt.%, of said non-crosslinked linear polyacrylic acid;
9 - 35 wt.% , preferably 15 - 32 wt.%, of said active pharmaceutical ingredient (API);
optionally, 0.01 - 10 wt.% of said one or several pharmaceutically acceptable excipient(s); or
b) 32 - 55 wt.% of said non-cross-linked linear polyacrylic acid;
5 - 25 wt.% of said active pharmaceutical ingredient (API); and
32 - 55 wt.% of said one or several pharmaceutically acceptable excipient(s); wherein, in both a) and b), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

10. The mucoadhesive layer according to any of claims 7 - 9, comprising the following wt.% proportions:
a) 94 - 97 wt.%, 92 - 93 wt.%, 89 - 91 wt.%, 87 - 88 wt.%, 84 - 86 wt.%, 82 - 83 wt.%, 79 - 81 wt.%, 77 - 78 wt.%, 74 - 76 wt.%, 72 - 73 wt.%, 69 - 71 wt.%, 67 - 68 wt.%, or 64 - 66 wt.% of said non-crosslinked linear polyacrylic acid; 3 - 6 wt.%, 7 - 8 wt.%, 9 - 11 wt.%, 12 - 13 wt.%, 14 - 16 wt.%, 17 - 18 wt.%, 19 - 21 wt.%, 22 - 23 wt.%, 24 - 26 wt.%, 27 - 28 wt.%, 29 - 31 wt.%, 32 - 33 wt.%, or 34 - 36 wt.% of said active pharmaceutical ingredient (API);
optionally, 0.01 - 10 wt.% of said one or several pharmaceutically acceptable excipient(s); or
b) 33 - 54 wt.% of said non-cross-linked linear polyacrylic acid;
10 - 20 wt.% of said active pharmaceutical ingredient (API);
33 - 54 wt.% of said one or several pharmaceutically acceptable excipient(s); wherein, in both a) and b), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

11. The mucoadhesive layer according to any of the foregoing claims, comprising the following weight proportions:
a) 82 - 84 wt.% of said non-cross-linked linear polyacrylic acid;
15 - 18 wt.% of said active pharmaceutical ingredient (API);
optionally, 0.01 - 3 wt.% of said one or several pharmaceutically acceptable excipient(s); or
b) 67 - 69 wt.% of said non-cross-linked linear polyacrylic acid;
30 - 33 wt.% of said active pharmaceutical ingredient (API);
optionally, 0.01 - 3 wt.% of said one or several pharmaceutically acceptable excipient(s); or
c) 52 - 54 wt.% of said non-cross-linked linear polyacrylic acid;
11 - 12 wt.% of said active pharmaceutical ingredient (API);
33 - 36 wt.% of said one or several pharmaceutically acceptable excipient(s); or
d) 33 - 36 wt.% of said non-cross-linked linear polyacrylic acid;
11 - 13 wt.% of said active pharmaceutical ingredient (API);
52 - 55 wt.% of said one or several pharmaceutically acceptable excipient(s);
wherein, in a), b), c) and d), wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

12. The mucoadhesive layer according to any of the foregoing claims, wherein said one or several pharmaceutically acceptable excipient(s) is(are) selected from a combination of: a) hydroxpropyl cellulose (HPC), polyvinylpyrrolidone (PVP), a plasticizer, such as glycerol, a colorant, and, optionally, a taste-masking or sweetening agent; and b) vinylpyrrolidone-vinylacetate-copolymer (e.g. Kollidon^{®}), a plasticizer, such as glycerol, a colorant, and, optionally, a taste-masking or sweetening agent.

13. The mucoadhesive layer according to any of claims 11c) and 12a), wherein said one or several pharmaceutically acceptable excipient(s) is(are) a combination of hydroxypropyl cellulose (HPC), polyvinylpyrrolidone (PVP), a plasticizer, such as glycerol, a colorant, and, optionally, a taste-masking or sweetening agent; wherein said mucoadhesive layer comprises 12 - 13 wt.% hydroxypropyl cellulose (HPC), 11 - 13 wt.% polyvinylpyrrolidone, 4 -5 wt.% plasticizer, 5 - 6 wt.% colorant and, optionally, 0.01 - 0.2 wt.% of a taste-masking or sweetening agent;
wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

14. The mucoadhesive layer according to any of claims 11d) and 12b), wherein said one or several pharmaceutically acceptable excipient(s) is(are) a combination of vinylpyrrolidone-vinylacetate-copolymer (e.g. Kollidon^{®}), a plasticizer, such as glycerol, a colorant, and, optionally, a taste-masking or sweetening agent; wherein said mucoadhesive layer comprises 42 - 45 wt.% vinylpyrrolidone-vinylacetate-copolymer (e.g. Kollidon^{®}), 4 - 5 wt.% plasticizer, 5 - 6 wt.% colorant and, optionally, 0.01 - 0.2 wt.% of a taste-masking or sweetening agent;
wherein wt.% is with reference to the total dry weight of said mucoadhesive layer;
with the proviso that the wt.% proportions of said non-crosslinked linear polyacrylic acid, said active pharmaceutical ingredient (API) and said one or several pharmaceutically acceptable excipient(s) in said mucoadhesive layer add up to 100 wt.%.

15. The mucoadhesive layer according to any of the foregoing claims, wherein said non-crosslinked linear polyacrylic acid has a molecular weight in the range of from 6kDa to 700 kDa, preferably from 100 kDa to 700 kDa, more preferably from 300 kDa to 700 kDa, even more preferably from 400 to 700 kDa, even more preferably from 500 to 700 kDa, even more preferably in the range of from 500 to 600 kDa.

16. The mucoadhesive layer according to any of the foregoing claims, having a thickness in the range of from 20 µm to 1500 µm, preferably 20 µm to 1000 µm, more preferably 20 µm to 800 µm, more preferably 30 µm to 600 µm, even more preferably 40 µm to 400 µm, yet even more preferably 50 µm to 200 µm. and most preferably 60 µm to 150 µm; wherein said thickness refers to a thickness of said mucoadhesive layer in a dry state; alternatively said mucoadhesive layer having a thickness in the range of from 20 µm to 1500 µm, preferably 20 µm to 1000 µm, more preferably 20 µm to 800 µm, more preferably 30 µm to 600 µm, even more preferably 40 µm to 500 µm yet even more preferably 90 µm to 450 µm, yet even more preferably 90 µm to 420 µm; wherein said thickness refers to a thickness of said mucoadhesive layer in a dry state.

17. The mucoadhesive layer according to any of the foregoing claims, said mucoadhesive layer comprising said active pharmaceutical ingredient (API) in an amount in the range of from 0.01 mg to 10 mg per cm² of mucoadhesive layer, or in an amount of 0.05 - 200 mg per g of mucoadhesive layer.

18. The mucoadhesive layer according to any of the foregoing claims, wherein said active pharmaceutical ingredient (API) is an immunosuppressant, wherein, preferably, said immunosuppressant is selected from tacrolimus, sirolimus, mycophenolic acid and its prodrugs.

19. A mucoadhesive film, preferably a mucoadhesive buccal film, comprising a mucoadhesive layer according to any of claims 1 - 18, and further comprising a backing layer attached to said mucoadhesive layer, wherein said backing layer, preferably, is permeable for water.

20. The mucoadhesive film according to claim 19, wherein said backing layer comprises a polymer selected from cellulose, cellulose derivatives, including hydroxypropyl cellulose (HPC), hydroxyethyl cellulose (HEC), hydroxypropylmethyl cellulose (HPMC), ethyl cellulose (EC), propyl cellulose (PC), and alginates, and wherein furthermore, said backing layer optionally comprises a plasticizer.

21. The mucoadhesive film according to any of claims 19 - 20, wherein said backing layer has a time-dependent dissolution in water or aqueous solutions, with a defined first dissolution time, and wherein said mucoadhesive layer also has a time-dependent dissolution in water or aqueous solutions with a defined second dissolution time, wherein said first dissolution time is smaller than, equal to or larger than said second dissolution time. ; wherein, preferably, said first dissolution time is in the range of from 1 min to 60 min, more preferably, in the range of from 1 min to 45 min, even more preferably in the range of from 1 min to 30 min.

22. A method of preparing a mucoadhesive layer, as defined in any of claims 1 - 18, said method comprising the steps:
a) Providing, in any order but separate from each other, an active pharmaceutical ingredient (API), a non-crosslinked linear polyacrylic acid, and, optionally, one or several pharmaceutically acceptable excipient(s), said API, said non-crosslinked linear polyacrylic acid and said one or several pharmaceutically acceptable excipient(s) being as defined in any of claims 1 - 18;
b) dissolving said active pharmaceutical ingredient (API), said non-crosslinked linear polyacrylic acid, and, optionally, said one or several pharmaceutically acceptable excipient(s) in a suitable organic or aqueous solvent, preferably selected from ethanol, acetone, isopropanol, dichloromethane (DCM), water and mixtures of any of the foregoing, thereby obtaining a viscous solution;
c) casting said viscous solution as a layer on a plane surface;
d) drying said casted layer, preferably at a temperature in the range of from 20 °C to 60 °C, more preferably 20 °C to 50 °C, wherein, optionally, said drying is performed under vacuum conditions;
e) releasing said dried layer from said plane surface, thereby obtaining a mucoadhesive layer as defined in any of claims 1 - 18.

23. A method of preparing a mucoadhesive film, as defined in any of claims 19 - 21, said method comprising the steps:
a') preparing, in any order, a mucoadhesive layer and a backing layer, wherein preparing said mucoadhesive layer is performed in accordance with claim 22;
b') laminating said backing layer to said mucoadhesive layer, under suitable conditions of temperature and/or pressure, such that said backing layer becomes attached to said mucoadhesive layer, and such that no air bubbles get trapped between said mucoadhesive layer and said backing layer.
